(19) Europäisches Patentamt
European Patent Office
Office européen des brevets

(11) Publication number: **0 070 133**
**B1**

(12) **EUROPEAN PATENT SPECIFICATION**

(45) Date of publication of patent specification: **14.05.86**

(21) Application number: **82303507.6**

(22) Date of filing: **05.07.82**

(51) Int. Cl.$^4$: **C 07 C 93/14,** C 07 C 103/38, C 07 C 121/34, C 07 C 125/065, C 07 C 127/15, C 07 C 143/822, C 07 D 295/08, C 07 D 307/81, A 61 K 31/135, A 61 K 31/16, A 61 K 31/27

# (54) Secondary phenylethanol amines, processes for their preparation and their pharmaceutical application.

(30) Priority: **11.07.81 GB 8121442**
**30.12.81 GB 8139024**

(43) Date of publication of application:
**19.01.83 Bulletin 83/03**

(45) Publication of the grant of the patent:
**14.05.86 Bulletin 86/20**

(84) Designated Contracting States:
**BE CH DE FR GB IT LI NL SE**

(56) References cited:
**EP-A-0 006 735**
**EP-A-0 007 204**
**EP-A-0 023 385**
**EP-A-0 029 320**
**EP-A-0 052 963**

(73) Proprietor: **BEECHAM GROUP PLC**
**Beecham House Great West Road**
**Brentford Middlesex TW8 9BD (GB)**

(72) Inventor: **Hindley, Richard Mark**
**19 Cockshot Road**
**Reigate Surrey (GB)**

(74) Representative: **Russell, Brian John et al**
**European Patent Attorney**
**Beecham Pharmaceuticals**
**Great Burgh Yew Tree Bottom Road**
**Epsom Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 070 133 B1

**Description**

The present invention relates to derivatives of ethanolamine which have anti-obesity and/or anti-hyperglycaemic and/or anti-inflammatory and/or platelet aggregation inhibition activity, to processes for their production and to their use in medicine.

European Patent Application No. 79301197.4 (Beechams) discloses compounds of formula

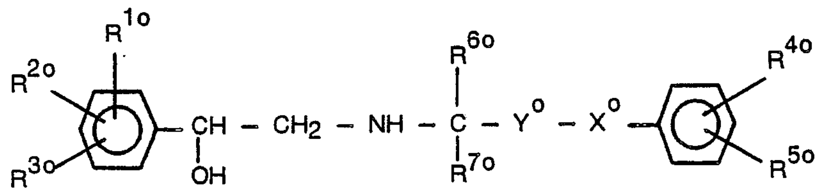

wherein

$R^{1o}$ is hydrogen, fluorine, chlorine, hydroxyl, hydroxymethyl, methyl, methoxy, amino, formamido, acetamido, methylsulphonamido, nitro, benzyloxy, methylsulphonylmethyl, ureido, trifluoromethyl or p-methoxybenzylamino;

$R^{2o}$ is hydrogen, fluorine, chlorine or hydroxyl;

$R^{3o}$ is hydrogen, fluorine, chlorine or hydroxyl;

$R^{4o}$ is a carboxylic acid group or a salt, ester or amide thereof;

$R^{5o}$ is hydrogen, fluorine, chlorine, methyl, methoxy, hydroxyl, or a carboxylic acid group or a salt, ester or amide thereof;

$R^{6o}$ is hydrogen, methyl, ethyl or propyl;

$R^{7o}$ is hydrogen, methyl, ethyl or propyl;

$X^o$ is oxygen or a bond; and

$Y^o$ is $C_{1-6}$ alkylene or a bond,

which possess anti-obesity and/or hypoglycaemic activity.

European Patent Application, Publication No. 0,023,385 discloses compounds of formula:

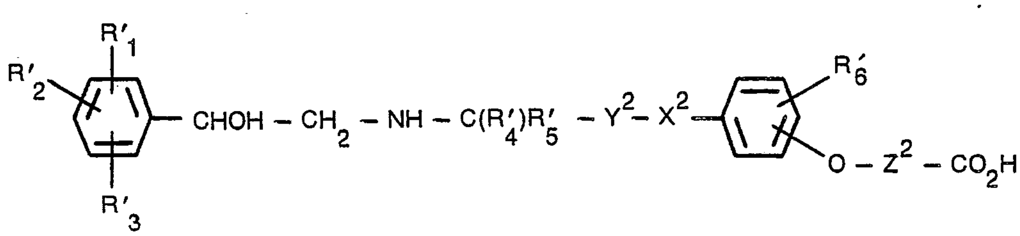

in which $R_1'$ is a hydrogen, fluorine, chlorine or bromine atom or a hydroxy, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group; $R_2'$ is a hydrogen, fluorine, chlorine or bromine atom or a hydroxyl group; $R_3'$ is a hydrogen, chlorine or bromine atom or a hydroxyl group, $R_4'$ is a hydrogen atom or a methyl group; $R_5'$ is a hydrogen atom or a methyl group; $R_6'$ is a hydrogen, fluorine or chlorine atom or a methyl, methoxyl or hydroxy group; $X^2$ is an oxygen atom or a bond; $Y^2$ is an alkylene group of up to 6 carbon atoms or a bond; and $Z^2$ is an alkylene, alkenylene or alkynylene group of up to 10 carbon atoms, which have been found to possess anti-obesity and/or anti-hyperglycaemic activity.

European Patent Application, Publication No. 0,007,204 discloses compounds of formula:

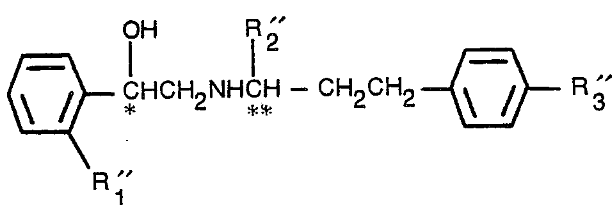

wherein

$R_1''$ is hydrogen or fluorine;

$R_2''$ is methyl or ethyl;

$R_3''$ is hydroxy, $C_1$—$C_4$ alkanoyloxy, aminocarbonyl, methylaminocarbonyl or $C_1$—$C_2$ alkoxycarbonyl;

C* is an asymmetric carbon atom having the R absolute stereochemical configuration; and C** is an

asymmetric carbon atom having the S absolute stereochemical configuration; and pharmaceutically acceptable salts thereof, which are useful in the weight control of mammals.

European Patent Application, Publication No. 0,029,320 discloses of compounds formula:

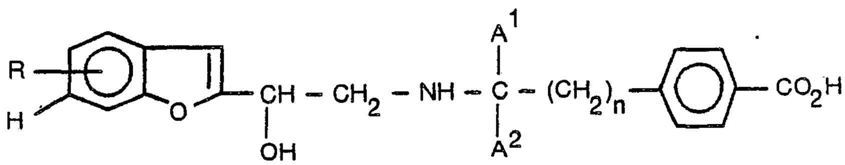

and esters, amides and pharmaceutically acceptable salts thereof, wherein

$A^1$ is hydrogen or methyl;

$A^2$ is hydrogen or methyl;

n is 1, 2 or 3; and

R is hydrogen, chlorine, bromine, hydroxy, nitro, amino or trifluoromethyl, which are useful as anti-hyperglycaemic agents and/or anti-obesity agents.

It has now been discovered that a class of novel phenoxyalkylaminoethanolamine derivatives have anti-obesity and/or anti-hyperglycaemic and/or anti-inflammatory and/or platelet aggregation inhibition activity. This activity is coupled with low cardiac stimulant activity for particular members of the class.

Accordingly, the present invention provides a compound of formula (I):

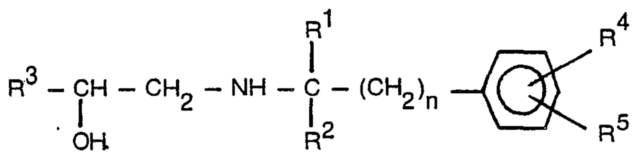

(I)

and salts and *in vivo* hydrolysable acyl derivatives thereof, wherein

$R^1$ is hydrogen or methyl;

$R^2$ is hydrogen or methyl;

$R^3$ is phenyl unsubstituted or substituted with one or two of the following:— fluorine, chlorine, bromine, trifluoromethyl, $C_{1-6}$ straight or branched chain alkyl or $C_{1-6}$ straight or branched chain alkoxy; or is benzofuran-2-yl;

$R^5$ is a hydrogen, halogen, hydroxy, lower alkyl or lower alkoxy;

$R^4$ is moiety

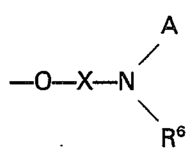

or

$—O—X^1—CH$

wherein X is $C_{2-10}$ alkylene which may be straight or branched provided that the oxygen and nitrogen are separated by at least two carbon atoms,

$X^1$ is $C_{1-9}$ alkylene which may be straight or branched,

A is hydrogen; $C_{1-6}$ straight or branched alkyl or benzyl optionally substituted with halogen, lower alkyl or lower alkoxy,

$R^6$ is hydrogen; $C_{1-6}$ straight or branched alkyl;. $C_{1-6}$ straight or branched alkylsulfonyl or

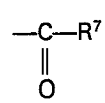

$R^7$ is $C_{1-6}$ straight or branched alkyl; $C_{1-6}$ straight or branched alkoxy; amino, mono- or di-($C_{1-6}$ straight or branched alkyl) amino; or

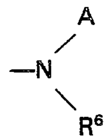

is a 5, 6 or 7 membered cyclic amino group optionally containing a second hetero atom selected from oxygen, nitrogen and sulphur, and

n is 1 or 2

Suitable cyclic amino groups

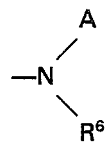

include pyrrolidine, piperidine, homopiperidine and morpholine groups.

Pharmaceutically acceptable salts of compounds of formula (I) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicyclic acid or acetylsalicylic acid.

The salts of compounds of formula (I) need not be pharmaceutically acceptable as they are also useful in the preparation of other compounds of formula (I) and in the separation of stereoisomers of compounds of formula (I) when the salt ion is also optically active.

*In vivo* hydrolysable acyl derivatives are those which are converted to compounds of formula (I) in the body of a patient to whom the acyl derivative has been administered.

Compounds of formula (I) have at least one asymmetric carbon atom, i.e. the carbon atom bearing the hydroxyl group and $R^3$ and, when $R^1$ and $R^2$ are different, the carbon atom bearing $R^1$ and $R^2$ is also asymmetric. The compounds may therefore exist in at least two and often four stereoisomeric forms. The present invention encompasses all stereoisomers of the compounds of formula (I) whether free from other stereoisomers or admixed with other stereoisomers in any proportion and thus includes, for instance, racemic mixtures of enantiomers.

Preferably, the carbon atom bearing the hydroxyl groups and $R^3$ has the R configuration.

The most potent compounds of formula (I) are those wherein $R^1$ and $R^2$ are different and both asymmetric carbon atoms are in the R configuration.

The absolute configuration of any compound of formula (I) may be determined by conventional X-ray crystallographic techniques.

It is believed that, in the $^{13}C$ n.m.r. spectrum of compounds of formula (I) wherein one of $R^1$ and $R^2$ is hydrogen and the other is methyl, the diastereoisomer having the greater anti-obesity and anti-hyperglycaemic activity is that for which the signal of the methyl group carbon atom appears at higher field (the lower numerical value when expressed in ppm) in $d_6$DMSO solution. The paired resonances often appear at approximately 20 ppm (less active) and slightly below 20 ppm (more active) down field from tetramethylsilane. Other paired resonances can occur for the carbon atoms attached directly to the nitrogen atom and the carbon which carries the hydroxyl group and $R^3$.

Again the paired resonances of the more active diastereoisomer of the investigated compounds appear at the higher field position.

The present invention provides a process for producing a compound of formula (I) or a salt, thereof, which process comprises reducing an oxo-group and/or a double bond and/or a moiety reducible to a group $R^4$ of a compound of formula (II):

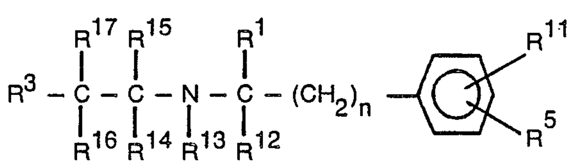

(II)

wherein

$R^1$, $R^3$, $R^5$ and n are as defined in relation to formuloa (I)

$R^{11}$ is $R^4$ or a moiety reducible to a group $R^4$ as defined in relation to formula (I);

$R^{12}$ is a group $R^2$ as defined in relation to formula (I) or together with $R^{13}$ forms a bond;
$R^{13}$ is hydrogen or benzyl; or together with $R^{12}$ or $R^{14}$ forms a bond;
$R^{14}$ is hydrogen or together with $R^{15}$ forms an oxo-group or together with $R^{13}$ forms a bond;
$R^{15}$ is hydrogen or together with $R^{14}$ forms an oxo-group;
$R^{16}$ is hydrogen or together with $R^{17}$ forms an oxo-group;
$R^{17}$ is hydrogen or together with $R^{16}$ forms an oxo-group
provided that there is no more than one oxo-group and no more than one bond represented by any of $R^{12}$ to $R^{17}$, and optionally thereafter forming a salt of the compound of formula (I) so formed and/or converting the compound of formula (I) so formed into a further compound of formula (I).

Where there are two or more reducible groups in the compound of formula (II) these may be reduced separately in any order or simultaneously.

The aforementioned reductions may be effected by conventional chemical or catalytic methods, such as chemical reduction using lithium aluminium hydride, sodium cyanoborohydride or sodium borohydride or boron methyl sulphide or by catalytic hydrogenation using catalysts such as palladium on charcoal, or platinum, for instance, as platinum oxide.

Reduction by sodium borohydride is conveniently effected in a lower alkanolic solvent such as methanol or ethanol. The reaction is generally carried out at from 0—20°C.

Reduction by lithium aluminum hydride is conveniently effected in a dry, ether solvent such as diethyl ether or tetrahydrofuran at ambient or elevated temperature.

Catalytic reduction is conveniently effected in a conventional hydrogenation solvent such as a lower alkanol, for instance ethanol., The hydrogenation is generally carried out under hydrogen gas at about 1 atmosphere pressure to about 10 atmosphere pressure and at ambient or elevated temperature.

In particular aspects, the present invention provides processes for producing compounds of formula (I) by reducing a compound of formula (IIA):

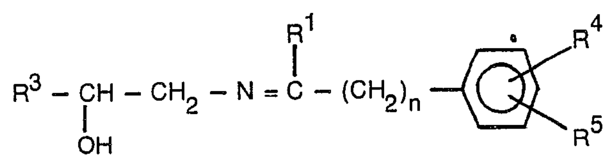

(IIA)

or reducing a compound of formula (IIB):

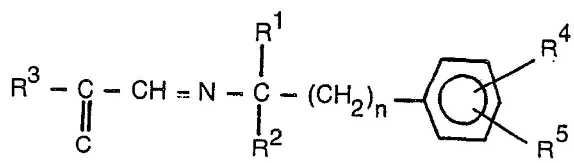

(IIB)

or reducing a compound of formula (IIC):

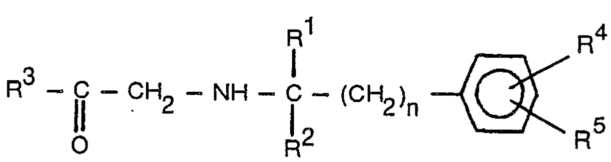

(IIC)

or the N-benzyl derivative thereof,
or reducing a compound of formula (IID):

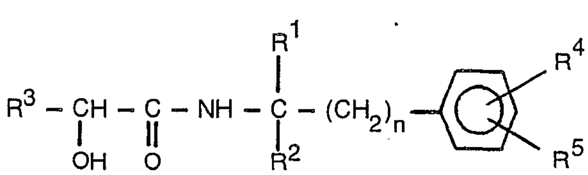

(IID)

or reducing a compound of formula (IIE):

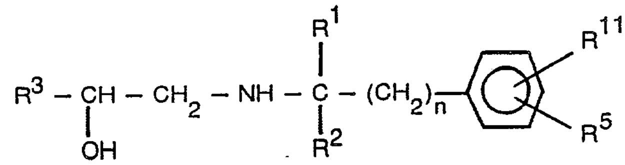

(IIE)

wherein $R^{11}$ is a moiety reducible to a group $R^4$, and

$R^1$, $R^2$, $R^3$, $R^4$, $R^5$ and n, are as defined in relation to formula (I).

Suitable groups for $R^{11}$ include cyanoalkoxy or amino carbonylalkoxy groups which may be reduced, for instance catalytically or using lithium aluminium hydride or boron methyl sulphide, to provide the corresponding aminoalkoxy group $R^4$.

A particular preferred process for producing compounds of formula (I) comprises the reduction of a compound of formula (IIA), especially using sodium borohydride in methanol at ambient temperature.

The present invention also provides another process for producing a compound of formula (I) or a salt thereof, which process comprises reacting a compound of formula (III):

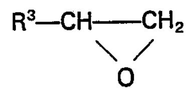

(III)

or a compound of formula (VIIID) as hereinafter defined with a compound of formula (IV):

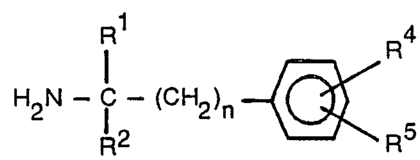

(IV)

wherein

$R^1$, $R^2$, $R^3$, $R^4$, $R^5$ and n, are as defined in relation to formula (I), and optionally thereafter forming a salt of the compound of formula (I) so formed.

The reaction of a compound of formula (III) with a compound of formula (IV) is conveniently effected in a solvent such as a lower alkanol, preferably ethanol.

The reaction of a compound of formula (VIIID) with a compound of formula (IV) is described below.

The present invention also provides yet another process for producing a compound of formula (I) or a salt thereof, which process comprises reacting an alkali metal salt of a compound of formula (V):

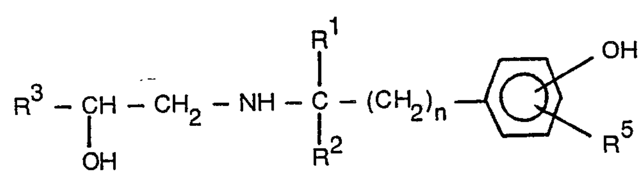

(V)

with a compound of formula (VI):

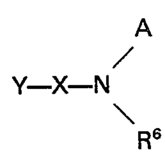

(VI)

wherein $R^1$, $R^2$, $R^3$, $R^5$, $R^6$, A, X and n are as defined in relation to formula (I).
and Y is a leaving group, such as halogen especially bromine, or tosyloxy.

The reaction of a compound of formula (V) with a compound of formula (VI) may be effected in a suitable solvent, which may be aqueous or organic, and preferably but not necessarily at elevated temperature.

Compounds of formula (I) may be converted into further compounds of formula (I) by conventional processes. Thus, for example, the compound of formula (I) wherein A and/or $R^6$ is hydrogen may be derivatised to produce further compounds of formula (I).

The salts of compounds of formula (I) may be produced by treating the compound of formula (I) with the appropriate acid.

Acyl derivatives of compounds of formula (I) may be produced by conventional methods.

Compounds of formula (I) and salts thereof, produced by the above processes, may be recovered by conventional methods.

Compounds of formula (I) having a single asymmetric carbon atom may, if desired, be separated into individual enantiomers by conventional means, for example by the use of an optically active acid as a resolving agent. Those compounds of formula (I) having two asymmetric carbon atoms may be separated into diastereoisomeric pairs of enantiomers by, for example, fractional crystallisation from a suitable solvent such as methanol or ethyl acetate of a mixture thereof. The pair of enantiomers thus obtained may be separated into individual stereoisomers by conventional means such as by the use of an optically active acid as a resolving agent.

Suitable optically active acids which may be used as resolving agents are described in "Topics in Stereochemistry" Vol. 6, Wiley Interscience, 1971, Allinger, N.L. and Eliel, W. L. Eds.

Alternatively any enantiomer of a compound of formula (I) may be obtained by stereospecific synthesis using an optically pure starting material of known configuration.

Compounds of formula (II) may be produced by reacting a compound of formula (VII):

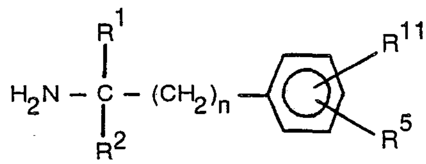

(VII)

wherein $R^1$, $R^2$, $R^5$ and n are as defined in relation to formula (I), and $R^{11}$ is as defined in relation to formula (II) with a compound of formula (VIII):

$$R^3—Y' \quad \text{(VIII)}$$

wherein $R^3$ is as defined in relation to formula (I) and Y' is a moiety containing a reactive group which is capable of reacting with the amine of formula (VII) thus forming a compound of formula (II). Typical examples of compounds of formula (VIII) are:

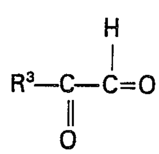

(VIIIA)

or its hydrate or hemi-acetal of a lower alkanol;

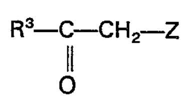

(VIIIB)

wherein Z is a halogen atom, preferably bromine;

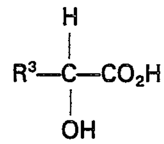 (VIIIC);

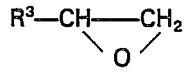 (III)

and

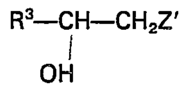 (VIIID)

wherein Z′ is a leaving group such as halogen or preferably tosylate.

Conventional conditions suitable for use with the particular compound of formula (VIII) may be used for this reaction. Thus the reaction of a compound of formula (VIIIA) with a compound of formula (VII) is conveniently conducted at elevated temperature under conditions resulting in the removal of the water formed during the reaction. A particularly suitable method is to perform the reaction in a solvent, such as benzene, under reflux and azeotropically to remove the water using a Dean and Stark trap.

The reaction of a compound of formula (VIIIB) with a compound of formula (VII) or its N-benzyl derivative is conveniently conducted in a polar organic solvent such as acetonitrile or butanone, at an elevated temperature, for instance under reflux.

The reaction of a compound of formula (VIIIC) with a compound of formula (VII) is conveniently conducted under standard peptide formation reaction conditions.

The reacton of a compound of formula (III) with a compound of formula (VII) is conveniently conducted in a solvent such as a lower alkanol, preferably ethanol.

The reaction of a compound of formula (VIIID) with a compound of formula (VII) is conveniently conducted in a solvent such as dimethylsulphoxide at elevated temperatures, preferably about 50°C for about 3 days.

When $R^{11}$ is a group $R^4$ the reaction between a compound of formula (III) or (VIIID) and a compound of formula (VII) give a compound of formula (I) directly and such processes form a further aspect of the present invention.

By using single enantiomers of a compound of formula (VII) and a compound of formula (VIII) such as the compounds (VIIIC) or (III) wherein $R^{11}$ is a moiety reducible to a group $R^4$ a stereospecific synthesis of a compound of formula (I) or (II) is achieved. The compound of formula (II) may then be reduced to a compound of formula (I) without altering the configuration of the two asymmetric carbon atoms. Thus, for example, a compound of formula (VII) with the *R*-absolute configuration and a compound of formula (III) with the *R*-absolute configuration would afford a compound of formula (I) or (II) with the *RR*-absolute configuration. Also a compound of formula (VII) with the *R*-absolute configuration and a compound of formula (VIIIC) of the *R*-absolute configuration would afford a compound of formula (II) and by subsequent reduction afford a compound of formula (I) with an *RR*-absolute configuration.

Certain compounds of formula (II) may also be produced by reacting a compound of formula (IX):

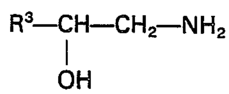 (IX)

with a compound of the formula (X):

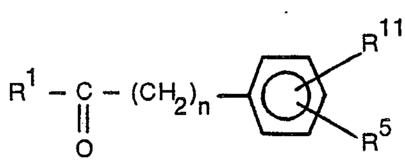 (X)

or with a compound of formula (XI):

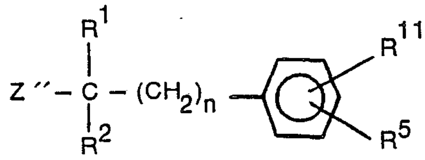

(XI)

wherein

$R^1$, $R^2$, $R^3$, $R^5$ and n are as defined in relation to formula (I);

$R^{11}$ is as defined in relation to formula (II); and

Z'' is a leaving group, suitably halogen or tosyloxy, preferably tosyloxy.

The reaction of a compound of formula (IX) with a compound of formula (X) is conveniently effected under conditions which result in the removal of water formed during the reaction. A particularly suitable method is to perform the reaction in a solvent, such as benzene, under reflux and azeotropically to remove the water using a Dean and Stark trap.

The reaction of a compound of formula (IX) with a compound of formula (XI) is conveniently effected in a solvent such as dimethylsulphoxide at elevated temperature, preferably about 50°C for about two to three days.

It is often convenient to prepare the compound of formula (II) and reduce it to the desired compound of formula (I) without isolation of the compound of formula (II).

A compound of formula (I) or a pharmaceutically acceptable salt thereof (hereinafter "the drug") may be administered as the pure drug, however, it is preferred that the drug be adminstered as a pharmaceutical composition also comprising a pharmaceutically acceptable carrier.

Accordingly, the present invention also provides a pharmaceutical composition comprising a compound of formula (I) or a pharmaceutically acceptable salt or *in vivo* hydrolysable acyl derivative thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed-unit dosage forms, such as powders presented in sachets, may also be used.

In accordance with conventional pharmaceutical practice the carrier may comprise a diluent, binder, filler disintegrant, wetting agent, lubricant, colourant, flavourant, or the like.

Typical carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides a method for treating obesity in humans or domestic mammals, which method comprises administering an effective, non-toxic amount of a compound of formula (I) or a pharmaceutically acceptable salt or *in vivo* hydrolysable acyl derivative thereof to obese humans or domestic mammals.

Conveniently, the drug may be administered as a pharmaceutical composition as hereinbefore defined, and this forms a particular aspect of the present invention.

In treating obese humans the drug may be taken in doses, such as those described above, one to six times a day in a manner such that the total daily dose for a 70 kg adult will generally be about 0.1 to 1000 mg, and more usually about 1 to 500 mg.

In treating obese domestic mammals, especially dogs, the drug may be administered by mouth, usually once or twice a day and at about 0.025 mg/kg to 2.5 mg/kg, for example 0.1 mg/kg to 2 mg/kg.

The present invention further provides a method for treating hyperglycaemia in humans which method comprises administering an effective, non-toxic amount of a compound of formula (I) or a pharmaceutically acceptable salt or *in vivo* hydrolysable acyl derivative thereof to hyperglycaemic humans.

The present invention also provides a method for the treatment of inflammation in humans, which comprises topically administering an effective non-toxic amount of a compound of formula (I) or a pharmaceutically acceptable salt thereof to humans suffering inflammation.

The present invention further provides a method of inhibiting platelet aggregation in humans, which comprises administering an effective non-toxic amount of a compound of formula (I) or a pharmaceutically acceptable salt thereof, to a human in need of such treatment.

Conveniently, the drug may be administered as a pharmaceutical composition as hereinbefore defined, and this forms a particular aspect of the present invention.

The drug may be taken in doses such as those described above for treating obese humans.

The invention will now be illustrated with reference to the following Examples, which are not intended to limit the scope in any way.

Example 1

N-[2-(4-(2-Methylaminoethoxy)phenyl)-1-methylethyl]-2-hydroxy-2-(3-trifluoromethylphenyl)ethanamine dihydrochloride

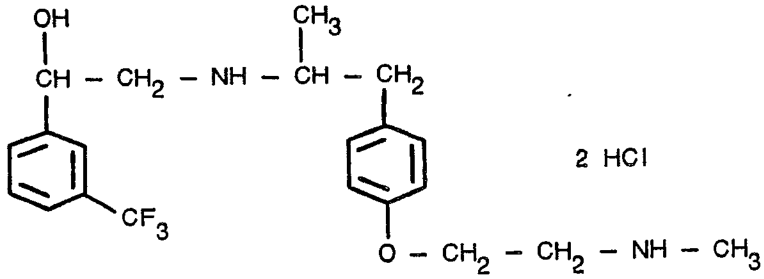

A mixture of 2-hydroxy-2-(3-trifluoromethylphenyl)ethanamine (3.0 g) and 1-[4-(2-methylaminoethoxy)phenyl]propan-2-one (3.0 g) in dry benzene was heated under reflux using a Dean and Stark head for two hours, cooled and evaporated to dryness. The residue was dissolved in methanol (50 ml) and hydrogenated over platinum oxide (50 mg) at normal temperature and pressure, filtered through diatomaceous earth and evaporated to dryness. Chromatography of the residue on silica gel in 5% methanol-dichloromethane gave an oil. Conversion to the dihydrochloride salt by passage of hydrogen chloride gas into an ethereal solution of the oil gave N-[2-(4-(2-methylaminoethoxy)phenyl)-1-methylethyl]-2-hydroxy-2-(3-trifluoromethylphenyl)ethanamine dihydrochloride. M.P. 214—216°C (methanol-ethylacetate) as a 73:27 mixture of diastereoisomers.

$^1$H nmr τ (DMSO-$d_6$)

8.9 (3H, d, J=6Hz), 7.4 (3H, s), 6.6—7.0 (7H, complex), 5.8 (2H, t), 4.8 (1H, m), 3.55 (1H, exchanges with $D_2O$), 3.05 (2H, d), 2.8 (2H, d), 2.15—2.45 (4H, complex), 0.65 (4H, exchanges with $D_2O$).

Example 1a

Recrystallisation of the above material twice from methanol gave a sample, m.p. 258—262°C, of 91:9 mixture of diastereoisomers.

Example 2

N-[2-(4-(2-Dimethylaminoethoxy)phenyl)-1-methylethyl]-2-hydroxy-2-(3-trifluoromethylphenyl)ethanamine dihydrochloride

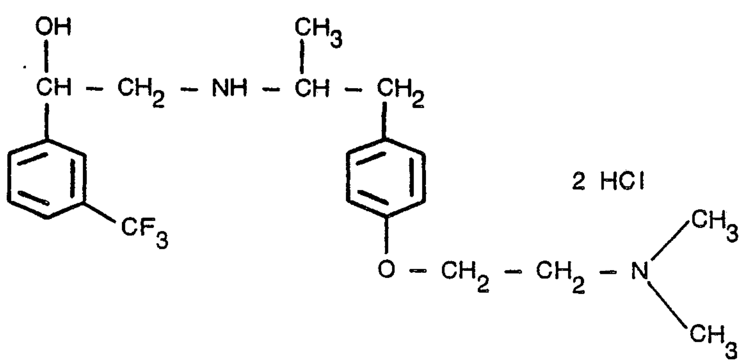

Starting from 2-hydroxy-2-(3-trifluoromethylphenyl)ethanamine (2.5 g) and 1-[4-(2-dimethylaminoethoxy)phenyl]propan-2-one (2.5 g) N-[2-(4-(2-dimethylaminoethoxy)phenyl)-1-methylethyl]-2-hydroxy-2-(3-trifluoromethylphenyl)ethanamine dihydrochloride, M.P. 218—223°C (methanol-ethylacetate), was prepared, as a 85:15 mixture of diastereoisomers by an analogous procedure to that described in Example 1.

$^1$H nmr τ (DMSO-$d_6$)

8.85 (3H, d), 7.15 (6H, s), 6.3—7.0 (7H, m), 5.6 (2H, t), 4.75 (1H, m), 3.5 (1H, exchanges with $D_2O$), 3.05 (2H, d), 2.75 (2H, d), 2.1—2.45 (4H, m), —0.5—1.0 (3H, broad; exchange with $D_2O$).

Example 3

N-[2-(4-(2-Methylsulphonylaminoethoxy)phenyl)-1-methylethyl]-2-hydroxy-2-(3-trifluoromethylphenyl)ethanamine

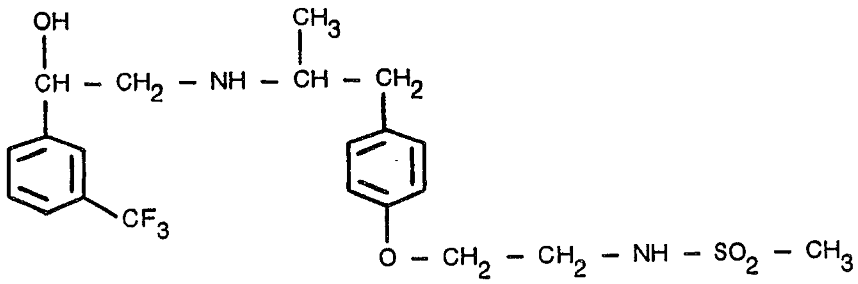

Starting from 2-hydroxy-2-(3-trifluoromethylphenyl)ethanamine (2.7 g) and 1-[4-(2-methylsulphonylaminoethoxy)phenyl)propan-2-one (3.2 g) N-[2-(4-(2-methylsulphonylaminoethoxy) phenyl)-1-methylethyl]-2-hydroxy-2-(3-trifluoromethylphenyl)ethanamine M.P. 96—100°C (diethyl ether-hexane) was prepared as a 56:44 mixture of diastereoisomers by an analogous procedure to that described in Example 1.

$^1$H nmr τ ($CDCl_3$ + $D_2O$)

8.95 (3H, d, J=6Hz), 7.0—7.6 (5H, complex), 7.05 (3H, s), 6.55 (2H, t), 5.95 (2H, t) 5.4 (1H, m), 3.2 (2H, d), 2.9 (2H, d), 2.3—2.6 (4H, complex).

Example 4

N-[2-(4-(2-Methylaminoethoxy) phenyl)-1-methylethyl]-2-hydroxy-2-(2-benzofuranyl) ethanamine dihydrochloride

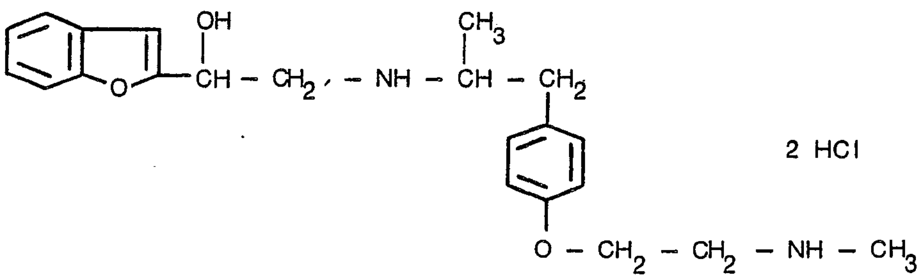

A mixture of 2-(2-benzofuranyl)-2-hydroxyethanamine (0.74 g) and 1-[4-(2-methylaminoethoxy) phenyl] propan-2-one (0.87 g) in benzene was heated under reflux using a Dean and Stark head for two hours, cooled, and evaporated to dryness. The residue was dissolved in methanol (60 ml) and sodium borohydride (1.0 g) was added portionwise, with stirring, at 0°C. The reaction mixture was stirred for one hour, then the methanol was evaporated and water was added to the residue, extracted with ethyl acetate (×2) dried (magnesium sulphate) and evaporated to dryness. The residue was purified by column chromatography on silica gel with 7% methanol in dichloromethane as eluent. The oil so obtained was converted to its dihydrochloride salt by addition of methanolic hydrogen chloride to give N-[2-(4-(2-Methylaminoethoxy) phenyl)-1-methylethyl]-2-hydroxy-2-(2-benzofuranyl)ethanamine dihydrochloride, m.p. 213—223°C (methanol-ethyl acetate) of analytical purity.

$^1$H nmr τ (DMSO-$d_6$)

8.85 (3H, d), 7.4 (3H, s), 6.25—7.0 (7H, complex), 5.7 (2H, t), 4.65 (1H, m), 3.4 (1H, exchanges with $D_2O$), 2.2—3 (9H, complex), 0.2—1.0 (4H, exchanges with $D_2O$).

Example 5

N-[2-(3-Fluoro-4-(2-methylaminoethoxy)phenyl)-1-methylethyl]-2-hydroxy-2-(3-chlorophenyl) ethanamine dihydrobromide

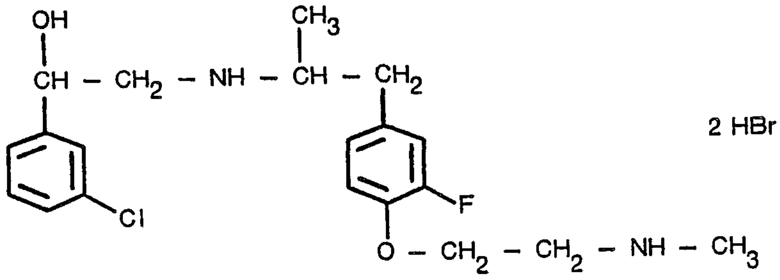

2 HBr

A mixture of 2-hydroxy-2-(3-chlorophenyl) ethanamine (1.3 g) and 1-[3-fluoro-4-(2-methylaminoethoxy) phenyl] propan-2-one (1.6 g) in dry benzene was heated under reflux using a Dean and Stark head for two hours, cooled and evaporated to dryness. The residue was dissolved in methanol, cooled with ice and sodium borohydride (2.0 g) was added in portions over fifteen minutes, with stirring; after stirring for a further hour, the solvent was evaporated. Water was then added to the residue and the residue extracted with ethyl acetate. The extract was dried (magnesium sulphate) and evaporated to dryness. Chromatography of the residue on silica gel eluting with 10% methanol in dichloromethane gave an oil which was converted to its dihydrobromide salt by addition of hydrogen bromide in methanol. Crystallisation from ethyl acetate gave N-[2-(3-Fluoro-4-(2-methylaminoethoxy)phenyl-1-methylethyl]-2-hydroxy-2-(3-chlorophenyl) ethanamine dihydrobromide, m.p. 207—214°, of analytical purity.

$^1$H nmr τ (DMSO-$d_6$)

8.85 (3H, d), 7.3 (3H, S), 6.3—7.2 (7H, m), 5.65 (2H, t), 4.9 (1H, m), 3.8 (1H, exchanges with $D_2O$), 2.4—3.1 (7H, complex), 1.1 (4H, exchanges with $D_2O$).

Example 6

N-[2-(4-(2-Methylaminoethoxy)phenyl)-1-methylethyl]-2-hydroxy-2-(3-ethylphenyl)ethanamine dihydrobromide

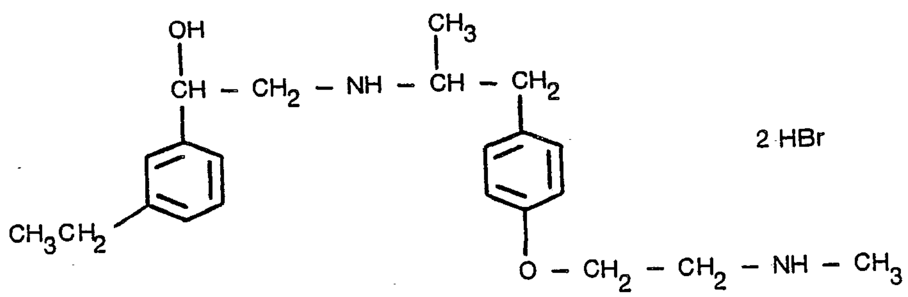

2 HBr

A mixture of 2-hydroxy-2-(3-ethylphenyl)ethanamine (2.39 g) and 1-[4-(2-methylaminoethoxy)phenyl]propan-2-one (3.0 g) in dry benzene was heated under reflux for 2 hours using a Dean and Stark head, cooled and the solvent evaporated. The residue was dissolved in ethanol and hydrogenated over platinum oxide at normal temperature and pressure, filtered through diatomaceous earth and evaporated to dryness. Chromatography of the residue on silica gel in methanol:chloroform:ammonia (8:92:0.5) gave an oil. Conversion to the dihydrobromide salt by passage of hydrogen bromide gas into an ethereal solution of the oil gave N-[2-(4-(2-methylaminoethoxy)phenyl)-1-methylethyl]-2-hydroxy-2-(3-ethylphenyl)ethanamine dihydrobromide m.p. 228—232°C (methanol-ethylacetate) as a 60:40 mixture of diastereoisomers.

$^1$H nmr τ (DMSO-$d_6$)

8.9 (3H, d), 8.8 (3H, t, J=7Hz), 7.4 (3H, s+2Hm), 6.3—7.0 (7H), 5.75 (2H, t), 5.0 (1H, m), 3.9 (1H, broad, disappears with $D_2O$), 3.2—2.65 (8H, m), 1.2 (4H, broad, disappears with $D_2O$).

Example 7

N-[2-(4-(2-Methylaminoethoxy)phenyl)-1-methylethyl]-2-hydroxy-2-(2-fluorophenyl)ethanamine dihydrobromide

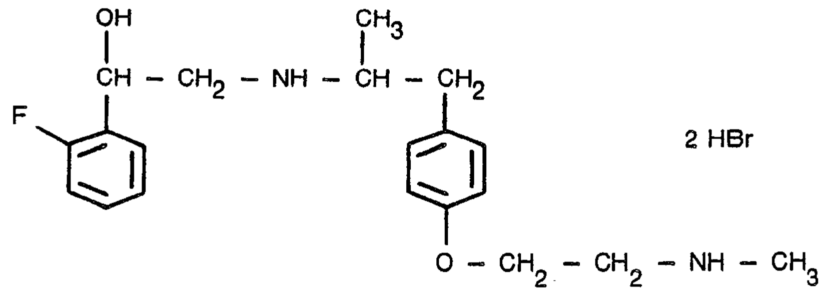

This was prepared in an identical manner to the compound described in Example 6 using 2-hydroxy-2-(2-fluorophenyl)ethanamine and 1-[4-(2-methylaminoethoxy)phenyl]propan-2-one. N-[2-(4-(2-methylaminoethoxy)phenyl)-1-methylethyl]-2-hydroxy-2-(2-fluorophenyl)ethanamine dihydrobromide m.p. 252—258°C (methanol-ethylacetate) was isolated as a 58:42 mixture of diastereoisomers.

$^1$H nmr τ (DMSO-$d_6$)

8.85 (3H, d), 7.5 (3H, s), 7.2—6.2 (7H, m), 5.85 (2H, t), 4.8 (1H, t), 3.1 (2H, d), 3.0—2.3 (6H, m), 1.7 (4H, broad, disappears with $D_2O$).

Example 8

N-[2-(4-(2-Methylaminoethoxy)phenyl)-1-methylethyl]-2-hydroxy-2-(3-bromophenyl)ethanamine dihydrobromide

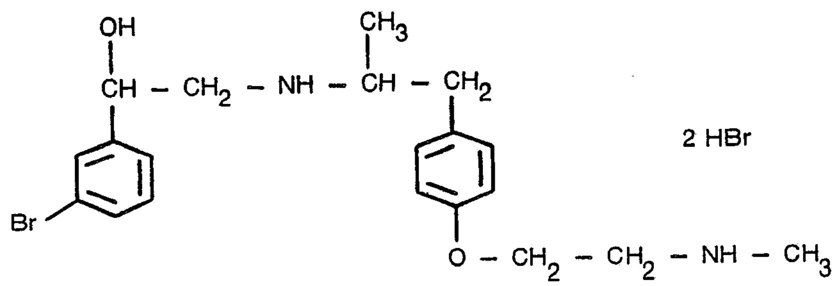

This was prepared in an identical manner to the compound described in Example 6 using 2-hydroxy-2-(3-bromophenyl)ethanamine and 1-[4-(2-methylaminoethoxy)phenyl]propan-2-one. N-[2-(4-(2-methylaminoethoxy)phenyl)-1-methylethyl]-2-hydroxy-2-(3-bromophenyl)ethanamine dihydrobromide m.p. 198—199°C (methanol-ethylacetate) was isolated as a 60:40 mixture of diastereoisomers.

$^1$H nmr τ (DMSO-$d_6$)

8.85 (3H, d), 7.35 (3H, s), 7.4—6.2 (7H, m), 5.72 (2H, t) 4.9 (1H, m), 3.7 (1H, broad, disappears with $D_2O$), 3.05 (2H, d), 2.8 (2H, m), 2.7—2.25 (4H, m), 1.2 (4H, broad, disappears with $D_2O$).

Example 9

N-[2-(4-(2-Methylaminoethoxy)phenyl-1-methylethyl]-2-hydroxy-2-(3-fluorophenyl)ethanamine dihydrobromide

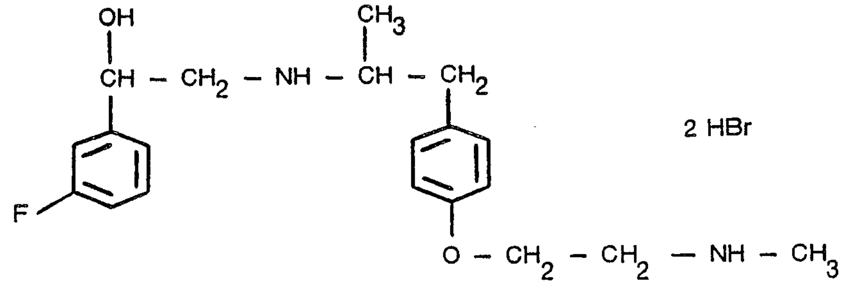

This compound was prepared in an analogous manner to that described in Example 5 using 2-hydroxy-2-(3-fluorophenyl)ethanamine and 1-[4-(2-methylaminoethoxy)phenyl]propan-2-one. Chromatography on silica gel eluting with methanol:chloroform:ammonia (8:92:1) gave an oil from which N-[2-(4-(2-methylaminoethoxy)phenyl)-1-methylethyl]-2-hydroxy-2-(3-fluorophenyl)ethanamine dihydrobromide m.p. 202—203°C (methanol-ethylacetate) was obtained as a 57:43 mixture of diastereoisomers.

$^1$H nmr τ (DMSO-$d_6$)
8.8 (3H, d), 7.3 (3H, s), 7.4—6.2 (7H, m), 5.7 (2H, t), 4.8 (1H, m), 3.75 (1H, disappears with $D_2O$), 3.0 (2H, d), 2.9—2.3 (6H, m), 1.1 (4H, disappears with $D_2O$).

Example 10

N-[2-(4-(2-Methylaminoethoxy)phenyl-1-methylethyl]-2-hydroxy-2-phenylethanamine dihydrobromide

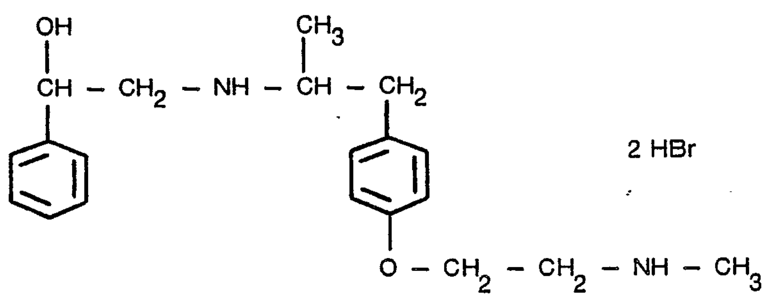

This was prepared in an identical manner to the compound described in Example 6 using 2-hydroxy-2-phenylethanamine and 1-[4-(2-methylaminoethoxy)phenyl]propan-2-one. N-[2-(4-(2-methylaminoethoxy)-phenyl)-1-methylethyl]-2-hydroxy-2-phenylethanamine (dihydrobromide m.p. 205—209°C (methanol-ethylacetate) was obtained as a 57:43 mixture of diastereoisomers.

$^1$H nmr τ (DMSO-$d_6$)
8.8 (3H, d), 7.3 (3H, s), 7.4—6.2 (7H, m), 5.75 (2H, t), 5.1 (1H, m), 3.5 (1H, disappears with $D_2O$), 3.05 (2H, d), 2.75 (2H, d), 2.5 (5H, m), 1.2 (4H, broad, disappears with $D_2O$).

Example 11

N-[2-(4-(2-Methylaminoethoxy) phenyl)-1-methylethyl]-2-hydroxy-2-(2-fluoro-3-chlorophenyl)ethanamine dihydrochloride

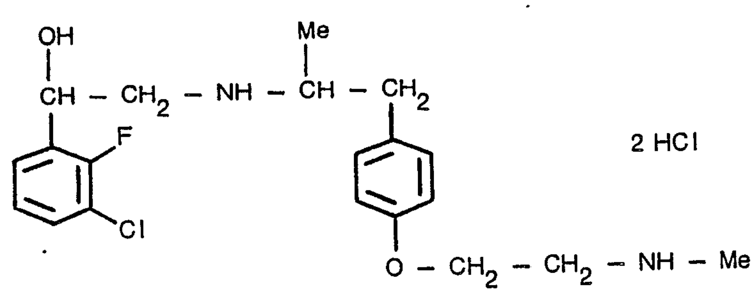

This was prepared in an identical manner to the comound described in Example 5 using 2 - hydroxy - 2 - (2 - fluoro - 3 - chlorophenyl ethanamine and 1 - [4 - (2 - methylaminoethoxy) - phenyl]propan - 2 - one. N - [2 - (4 - (2 - Methylaminoethoxy)phenyl - 1 - methlethyl] - 2 - hydroxy - 2 - (2 - fluoro - 3 - chlorophenyl) ethanamine dihydrochloride, m.p. 232—234°C (methanol-ethyl acetate), was isolated as a 49.51 mixture of diastereoisomers.

$^1$H nmr τ (DMSO-$d_6$)
8.8 (3H, d), 7.4 (3H, s), 6.3—7.4 (7H, m), 5.8 (2H, t), 4.5 (1H, m), 3.45 (1H, d, disappears with $D_2O$), 3.0 (2H, d), 2.75 (2H, d), 2.2—2.75 (3H, m), 0.5 (4H, broad, disappears with $D_2O$).

Example 12

N-[2-(4-(2-Methylaminoethoxy)phenyl)-1-methylethyl]-2-hydroxy-2-(4-chlorophenyl)ethanamine dihydrochloride

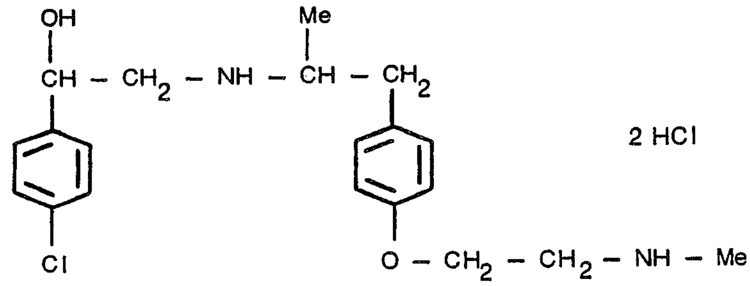

2 HCl

This was prepared in an identical manner to the compound described in Example 5 using 2 - hydroxy - 2 - (4 - chlorophenyl)ethanamine and 1 - [4 - (2 - methylaminoethoxy)phenyl] propan - 2 - one. N - [2 - (4 - (2 - Methylaminoethoxy)phenyl) - 1 - methylethyl] - 2 - hydroxy - 2 - (4 - chlorophenyl)ethanamine dihydrochloride, m.p. 232—246°C (methanol-ethyl acetate) was isolated as 60:40 mixture of diastereoisomers.

$^1$H nmr τ (DMSO-$d_6$)

8.8 (3H, d), 7.4 (3H, s), 6.6—7.4 (7H, m), 5.8 (2H, t), 4.8 (1H, m), 3.7 (1H, broad, disappears with $D_2O$), 3.1 (2H, d), 2.8 (2H, d), 2.5 (4H, s), 0.6 (4H, broad, disappears with $D_2O$).

Example 13

N-[2-(4-(2-Benzylaminoethoxy)phenyl)-1-methylethyl]-2-hydroxy-2-(3-chlorophenyl)ethanamine dihydrochloride monohydrate

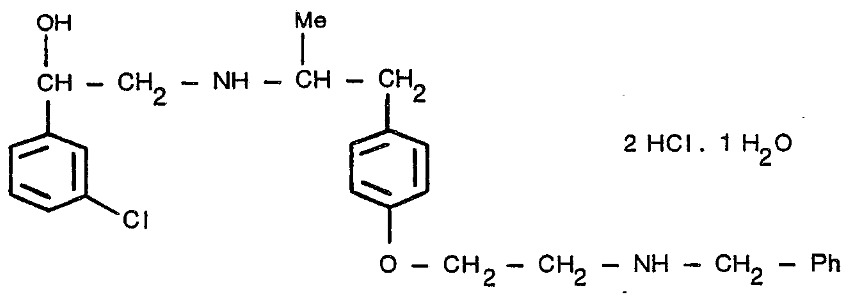

2 HCl . 1 $H_2O$

This was prepared in an identical manner to the compound described in Example 5 using 2 - hydroxy - 2 - (3 - chlorophenyl)ethanamine and 1 - [4 - (2 - benzylaminoethoxy)phenyl]propan - 2 - one. N - [2 - (4 - (2 - Benzylaminoethoxy)phenyl) - 1 - methylethyl] - 2 - hydroxy - 2 - (3 - chlorophenyl)ethanamine dihydrochloride monohydrate, m.p. 217—222°C (methanol-ethyl acetate) was isolated as a 47:53 mixture of diastereoisomers.

$^1$H nmr τ (DMSO-$d_6$)

8.8 (3H, d), 6.6—7.4 (7H, m), 5.8 (2H, s), 5.7 (2H, t), 4.9 (1H, m), 3.7 (1H, broad, disappears with $D_2O$), 3.1 (2H, d), 2.8 (2H, d), 2.3—2.7 (9H, m), 1.1 (1H, broad, disappears with $D_2O$), 0.2 (3H, broad, disappears with $D_2O$).

Example 14

N-[2-(4-(2-(1-Piperidine)ethoxy)phenyl)-1-methylethyl]-2-hydroxy-2-(3-chlorophenyl)ethanamine dihydrochloride

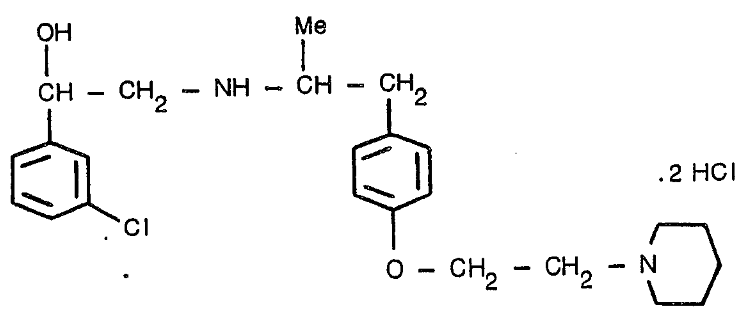

.2 HCl

This was prepared in an identical manner to the compound described in Example 5 using 2 - hydroxy - 2 - (3 - chlorophenyl)ethanamine and 1 - [4 - (2 - (1 - piperidine)ethoxy)phenyl]propan - 2 - one. N - [2 - (4 - (2 - (1 - Piperidine)ethoxy)phenyl] - 1 - methylethyl] - 2 - hydroxy - 2 - (3 - chlorophenyl)-ethanamine dihydrochloride, mp 211—213°C (methanol-ethyl acetate) was isolated as a 45:55 mixture of diastereoisomers.

$^1$H nmr τ(DMSO-$d_6$)

8.8 (3H, d), 8.0—8.7 (6H, m), 6.2—7.4 (11H, m), 5.55 (2H, t), 4.8 (1H, m), 3.55 (1H, broad, disappears with $D_2O$), 3.0 (2H, d), 2.75 (2H, d), 2.4—2.6 (4H, m), 0.95 (1H, broad, disappears with $D_2O$), 0.2 (1H, broad, disappears with $D_2O$), −1.5 (1H, broad, disappears with $D_2O$).

Example 15

N-[2-(4-(2-(1-Homopiperidine)ethoxy)phenyl)-1-methylethyl]-2-hydroxy-2-(3-trifluoromethylphenyl)-ethanamine dihydrochloride

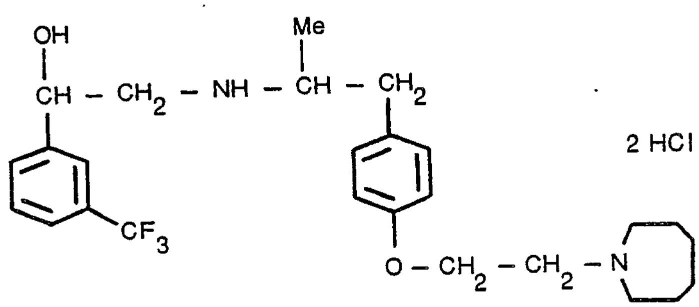

This was prepared in an identical manner to the compound described in Example 5 using 2 - hydroxy - 2 - (3 - trifluoromethyl)phenylethanamine and 1 - [4 - (2 - (1 - homopiperidine)ethoxy)phenyl]propan - 2 - one. N - [2 - (4 - (2 - (1 - Homopiperidine)ethoxy)phenyl) - 1 - methylethyl] - 2 - hydroxy 2 - (3 - trifluoromethylphenyl)ethanamine dihydrochloride, mp 179—183°C (methanol-ethyl acetate) was isolated as a 40:60 mixture of diastereoisomers.

$^1$H nmr τ(DMSO-$d_6$)

8.8 (3H, d), 8.0—8.6 (8H, m), 6.3—7.4 (11H, m), 5.55 (2H, t), 4.75 (1H, m), 3.5 (1H, broad, disappears with $D_2O$), 3.0 (2H, d), 2.75 (2H, d), 2.05—2.35 (4H, m), 0.95 (1H, broad, disappears with $D_2O$), 0.15 (1H, broad, disappears with $D_2O$), −1.9 (1H, broad, disappears with $D_2O$).

Example 16

N-[2-(4-(2-Ethoxycarbonylamioethoxy)phenyl)-1-methylethyl]-2-hydroxy-2-(3-chlorophenyl)ethanamine

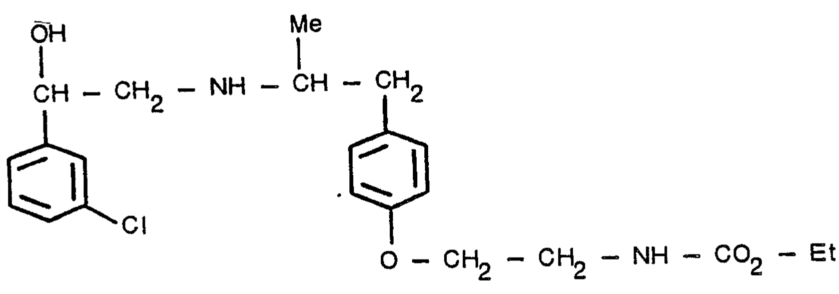

This was prepared in an identical manner to the compound described in Example 5 using 2 - hydroxy - 2 - (3 - chlorophenyl)ethanamine and 1 - [4 - (2 - ethoxycarbonylaminoethoxy)phenyl]propan - 2 - one. N - [2 - (4 - (2 - Ethoxycarbonylaminoethoxy)phenyl) - 1 - methylethyl] - 2 - hydroxy - 2 - (3 - chlorophenyl)ethanamine, mp 99—106°C (ether hexane) as a 40:60 mixture of diastereoisomers.

$^1$H nmr τ($CDCl_3$)

8.9 (3H, d), 8.8 (3H, t), 6.9—7.7 (6H, m, 1H, disappears with $D_2O$), 6.45 (1H, dt, collapses to t with $D_2O$), 6.0 (2H, t), 5.9 (2H, q), 5.55 (1H, m), 4.85 (1H, broad, disappears with $D_2O$), 3.15 (2H, d), 2.95 (2H, d), 2.8—2.6 (4H, m).

Example 17

N-[2-(4-(2-Methylaminocarbonylaminoethoxy)phenyl)-1-methylethyl]-2-hydroxy-2-(3-trifluoromethylphenyl)ethanamine

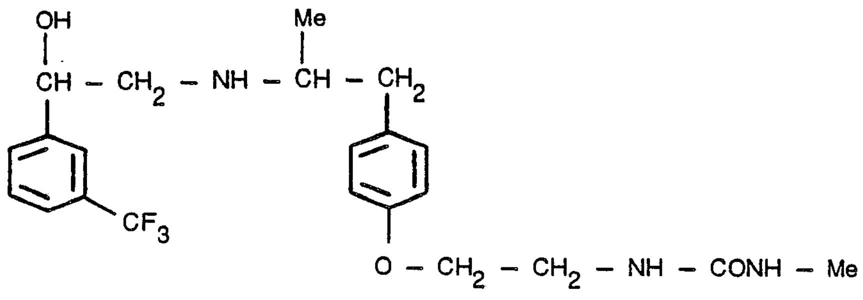

This was prepared in an identical manner to the compound described in Example 5 using 2 - hydroxy - 2 - (3 - trifluoromethylphenyl)ethanamine and 1 - [4 - (2 - methylaminocarbonylaminoethoxy)phenyl]-propan - 2 - one, N - [2 - (4 - (2 - Methylamino)carbonylaminoethoxy)phenyl - 1 - methylethyl] - 2 - hydroxy - 2 - (3 - trifluoromethylphenyl)ethanamine was isolated as the free base, mp 131—139°C (ethylacetate) as a 15:85 mixture of diastereoisomers.

$^1$H nmr τ(DMSO-$d_6$)

9.1 (3H, d), 7.8—8.7 (1H, broad), 7.45 (3H, d), 7.0—7.4 (5H, m), 6.65 (2H, dt), 6.1 (2H, t), 5.3 (1H, m), 4.5 (1H, broad), 4.15 (1H, q), 3.9 (1H, t), 3.15 (2H, d), 2.9 (2H, d), 2.2—2.6 (4H, m).

Example 18

N-[2-(4-(2-Benzylaminoethoxy)phenyl)-1-methylethyl]-2-hydroxy-2-(3-trifluoromethylphenyl)ethanamine

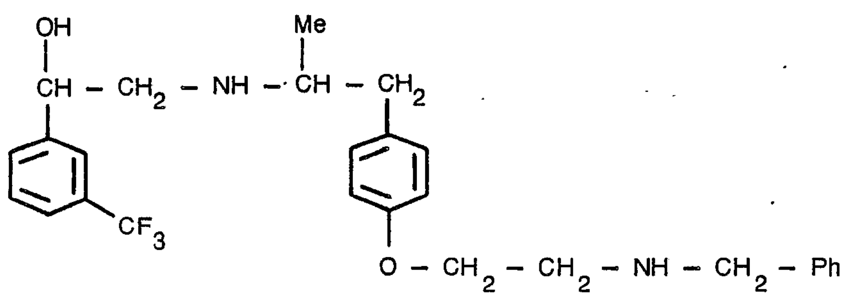

This was prepared in an identical manner to the compound described in Example 5 using (2 - hydroxy - 2 - (3 - trifluoromethylphenyl)ethanamine and 1 - [4 - (2 - benzylaminoethoxy)phenyl)-propan - 2 - one. Column chromatography on silica with methanol:dichloromethane (5:95) as eluent gave an oil which was crystallized from ethylacetate - hexane to give N - [2 - (4 - (2 - benzylaminoethoxy)-phenyl - 1 - methylethyl] - 2 - hydroxy - 2 - (3 - trifluoromethylphenyl)ethanamine, mp 67—81°C.

$^1$H nmr τ($CDCl_3$)

8.9 (3H, d), 6.9—7.6 (10H, complex, 3H disappears with $D_2O$), 6.15 (2H, s), 5.95 (2H, t), 5.3 (1H, m), 3.25 (2H, d), 2.95 (2H, d), 2.25—2.7 (9H, complex).

Example 19

N-[2-(4-(2-Aminoethoxy)phenyl)-1-methylethyl]-2-hydroxy-2-(3-trifluoromethylphenyl)ethanamine dihydrochloride, dihydrate

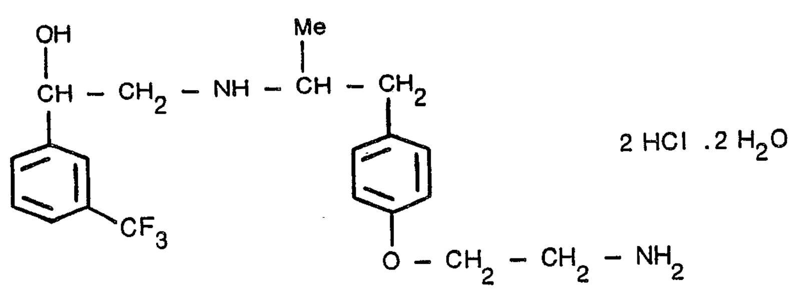

The compound of Example 18 (0.95 g) in ethanol (30 ml) containing palladium on charcoal (10% w/w) was hydrogenated at ambient temperature and pressure until hydrogen uptake ceased. The mixture was filtered through diatomaceous earth and evaporated to give N - [2 - (4 - (2 - aminoethoxy)phenyl) - 1 - methylethyl] - 2 - hydroxy - 2 - (3 - trifluoromethylphenyl)ethanamine dihydrochloride, dihydrate, mp 223—229°C (methanol-ethyl acetate), as a 61:39 mixture of diastereoisomers.

$^1$H nmr τ(DMSO-$d_6$ + $D_2O$)

8.9 (3H, d), 6.5—7.6 (complex), 4.8 (1H, m), 3.2 (2H, d), 2.8 (2H, d), 2.2 (4H, complex).

Example 20

N-[2-(4-(2-Ethoxycarbonylaminoethoxy)phenyl)-1-methylethyl]-2-hydroxy-2-(3-trifluoromethylphenyl)-ethanamine

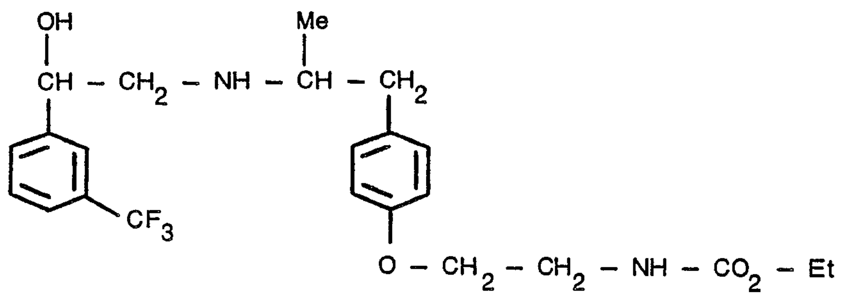

This was prepared in an identical manner to the compound described in Example 6 using 2 - hydroxy - 2 - (3 - trifluoromethylphenyl)ethanamine and 1 - [4 - (2 - ethoxycarbonylamineoethoxy)phenyl]-propan - 2 - one. Chromatography on silica gel using methanol-dichloromethane (5:95) as eluent gave an oil which crystallized to give N - [2 - (4 - (2 - ethoxycarbonylaminoethoxy)phenyl) - 1 - methylethyl] - 2 - hydroxy - 2 - (3 - trifluoromethylphenyl)ethanamine, mp 84—87°C (ether-hexane), as a 91:9 mixture of diastereoiosomers.

$^1$H nmr τ($CDCl_3$)

8.95 (3H, d), 8.8 (3H, t), 6.9—7.65 (7H, 2H disappears with $D_2O$), 6.5 (2H, dt), 5.8—6.2 (4H, complex), 5.4 (1H, m), 5.0 (1H, disappears with $D_2O$), 3.25 (2H, d), 2.95 (2H, d), 2.5 (4H, complex).

Example 21

N-[2-(4-(2-Acetylaminoethoxy)phenyl)-1-methylethyl]-2-hydroxy-2-(3-trifluoromethylphenyl)ethanamine

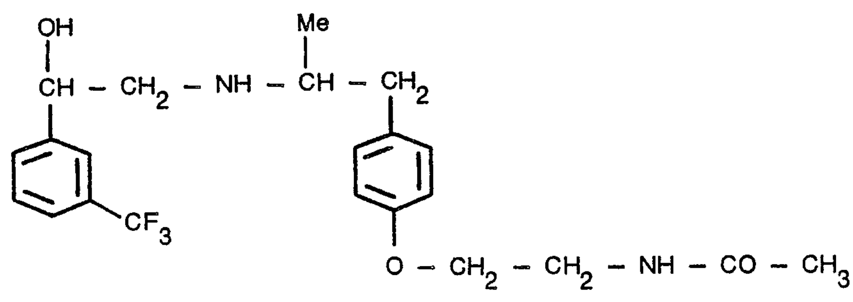

This was prepared in an identical manner to the compound described in Example 6 using 2 - hydroxy - 2 - (3 - trifluoromethylphenyl)ethanamine and 1 - [4 - (2 - acetylaminoethoxy)phenyl]propan - 2 - one. Chromatography on silica gel using methanol-dichloromethane (5:95) as eluent gave N - [2 - (4 - (2 - acetylaminoethoxy)phenyl) - 1 - methylethyl] - 2 - hydroxy - 2 - (3 - trifluoromethylphenyl)ethanamine, mp 78—84°C (ethyl acetate-ether), as a 43:57 mixture of diastereoisomers.

$^1$H nmr τ($CDCl_3$)

8.95 (3H, d), 8.05 (3H, s), 6.9—7.65 (7H, complex, 2H disappears with $D_2O$), 6.4 (2H, dt), 6.05 (2H, t), 5.4 (1H, m), 3.95 (1H, broad, disappears with $D_2O$), 3.25 (2H, d), 2.95 (2H, d), 2.45 (4H, m).

Example 22

N-[2-(4-(2-Methylaminoethoxy)phenyl)-1-methylethyl]-2-hydroxy-2-(3-chlorophenyl)ethanamine dihydrochloride

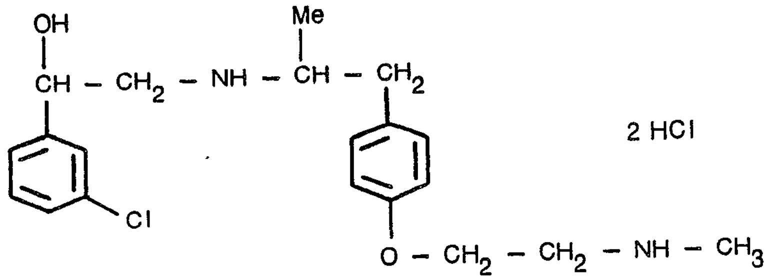

2 HCl

This was prepared in an identical manner to the compound described in Example 6 using 2 - hydroxy - 2 - (3 - chlorophenyl)ethanamine and 1 - [4 - (2 - methylaminoethoxy)phenyl]propan - 2 - one. N - [2 - (4 - (2 - Methylaminoethoxy)phenyl) - 1 - methylethyl] - 2 - hydroxy - 2 - (3 - chlorophenyl)ethanamine dihydrochloride, mp 220—226°C (methanol-ethylacetate), was isolated as a 58:42 mixture of diastereoisomers.

$^1$H nmr τ(DMSO-$d_6$)

8.85 (3H, d), 7.4 (3H, s), 6.5—7.3 (7H, complex), 5.8 (2H, t), 4.85 (1H, m), 3.65 (1H, disappears with $D_2O$), 3.05 (2H, d), 2.8 (2H, d), 2.4—2.6 (4H, complex), 0.65 (4H, disappears with $D_2O$).

Example 23

N-[2-(4-(2-Methylaminoethoxy)phenyl)-1-methylethyl]-2-hydroxy-2-(3-trifluoromethylphenyl)ethanamine dihydrochloride, (RS,SR) diastereoisomer

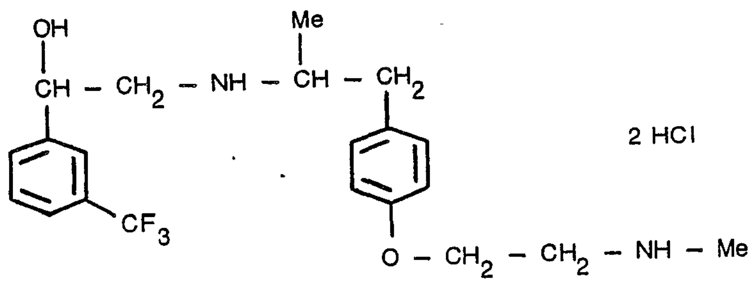

2 HCl

To a solution of the (RS,SR) diastereoisomer of N - [2 - (4 - (2 - methylaminocarbonylmethoxy)-phenyl - 1 - methylethyl] - 2 - hydroxy - 2 - (3 - trifluoromethylphenyl)ethanamine (2.48 g) in dry tetrahydrofuran (50 ml) was added borane-methylsulphide (5 ml) dropwise under nitrogen. The mixture was stirred at ambient temperature for 20 min and then heated under reflux for 4.5 h. After cooling a solution of methanol (15 ml) in tetrahydrofuran (30 ml) was added dropwise over 0.5 h. The solution was allowed to stand overnight at room temperature, heated under reflux for 1.5 h, cooled and hydrogen chloride gas passed through the solution. Ether (50 ml) was added and the solid filtered to give N - [2 - (4 - (2 - methylaminoethoxy)phenyl) - 1 - methylethyl] - 2 - hydroxy - 2 - (3 - trifluoromethylphenyl)ethanamine dihydrochloride, (RS,SR) diastereoisomer, mp 237—240°C, as a <5:>95 mixture of (RR,SS):(RS:SR) diastereoisomers.

$^1$H nmr

Identical with that of Example 1.

Example 24

N-[2-(4-(2-Methylaminoethoxy)phenyl)-1-methylethyl]-2-hydroxy-2-(3-trifluoromethylphenyl)ethanamine dihydrochloride (RR,SS) diastereoisomer

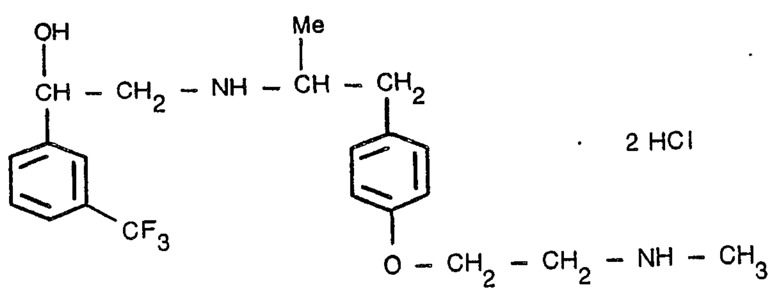

2 HCl

Starting with the (RR,SS) diastereoisomer of N - [2 - (4 - (2 - methylaminocarbonylmethoxy)-phenyl) - 1 - methylethyl] - 2 - hydroxy - 2 - (3 - trifluoromethylphenyl)ethanamine and following the procedure described in Example 23, N - [2 - (4 - (2 - methylaminoethoxy)phenyl) - 1 - methylethyl] - 2 - hydroxy - 2 - (3 - trifluoromethylphenyl)ethanamine dihydrochloride, (RR,SS) diastereoisomer, mp 263—266°C was obtained as a 97:3 mixture of (RR,SS):(RS,SR) diastereoisomers.

$^1H$ nmr

Identical with that of Example 1.

Example 25

N-[2-(4-(2-(1-Pyrrolidine)ethoxy)phenyl)-1-methylethyl]-2-hydroxy-2-(3-trifluoromethylphenyl)ethanamine dihydrochloride

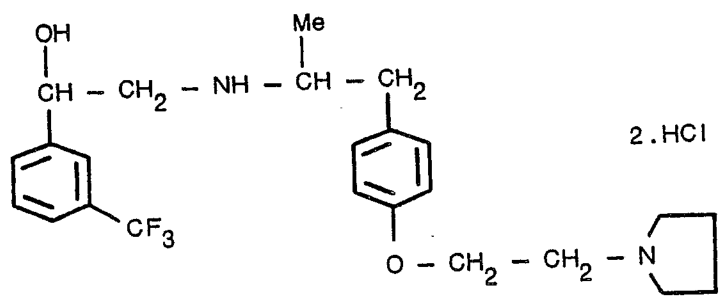

2 . HCl

This was prepared in an identical manner to the compound described in Example 5 using 2 - hydroxy - 2 - (3 - trifluoromethylphenyl)ethanamine and 1 - [4 - (2 - (1 - pyrrolidine)ethoxy)phenyl]propan - 2 - one. N - [2 - (4 - (2 - (1 - Pyrrolidine)ethoxy)phenyl) - 1 - methylethyl] - 2 - hydroxy - 2 - (3 - trifluoromethylphenyl)ethanamine dihydrochloride, mp 214—231°C (methanol-ethyl acetate) was isolated as a 17:83 mixture of diastereoisomers.

$^1H$ nmr $\tau$(DMSO-$d_6$)

8.8 (3H, d), 8.1 (4H, broad s), 7.7—6.3 (11H, complex), 5.65 (2H, t), 4.75 (1H, m), 3.55 (1H, broad, disappears with $D_2O$), 3.1 (2H, d), 2.8 (2H, d), 2.5—2.1 (4H, m), 0.9, 0.15 −1.5 (3H, all disappear with $D_2O$).

Example 26

N-[2-(4-(2-(1-Piperidine)ethoxy)phenyl)-1-methylethyl]-2-hydroxy-2-(3-trifluoromethylphenyl)ethanamine dihydrochloride monohydrate

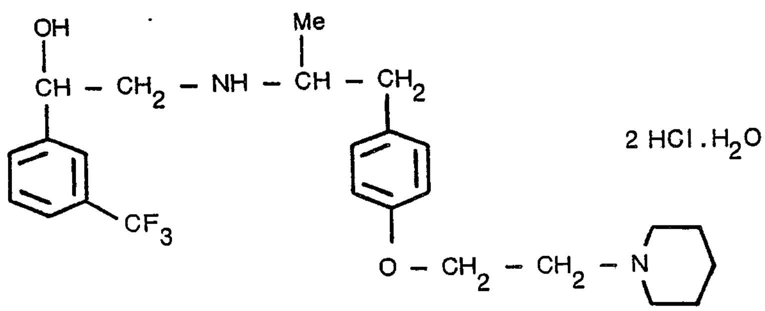

2 HCl . $H_2O$

This was prepared in an identical manner to the compound described in Example 5 using 2 - hydroxy - 2 - (3 - trifluoromethylphenyl)ethanamine and 1 - [4 - (2 - (1 - piperidine)ethoxy)phenyl]propan - 2 - one. N - [2 - (4 - (2 - (1 - Piperidine)ethoxy)phenyl) - 1 - methylethyl] - 2 - hydroxy - 2 - (3 - trifluoromethylphenyl)ethanamine dihydrochloride monohydrate, mp 176—185°C (methanol-ethyl acetate) was isolated as a 55:45 mixture of diastereoisomers.

$^1H$ nmr $\tau$(DMSO-$d_6$)

8.8 (3H, d), 8.7—7.9 (6H, complex), 7.5—6.3 (11H, complex), 5.5 (2H, t), 4.7 (1H, m), 3.5 (1H, disappears with $D_2O$), 3.1 (2H, d), 2.75 (2H, d), 2.5—2.1 (4H, m), 0.8, 0.0, −1.5 (3H, all disappear with $D_2O$).

Example 27

N-[2-(4-(2-Ethylaminoethoxy)phenyl)-1-methylethyl]-2-hydroxy-2-(3-trifluoromethylphenyl)ethanamine dihydrochloride

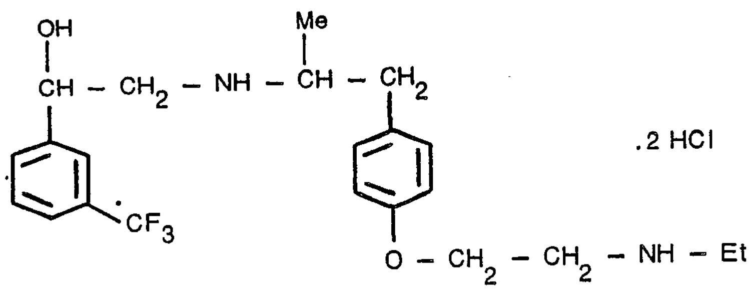

This was prepared in an analogous manner to the compound described in Example 23 starting from N - [2 - (4 - (2 - ethylaminocarbonylmethoxy)phenyl) - 1 - methylethyl] - 2 - hydroxy - 2 - (3 - trifluoromethylphenyl)ethanamine. N - [2 - (4 - (2 - Ethylaminoethoxy)phenyl) - 1 - methylethyl] - 2 - hydroxy - 2 - (3 - trifluoromethylphenyl)ethanamine dihydrochloride, mp 237—242°C (methanol-ethyl acetate) was isolated as a 66:34 mixture of diastereoisomers.

$^1$H nmr τ(DMSO-$d_6$)

8.9 (3H, d), 8.8 (3H, t), 7.5—6.3 (9H, complex), 5.75 (2H, t), 4.75 (1H, m), 3.5 (1H, disappears with $D_2O$), 3.05 (2H, d), 2.75 (2H, d), 2.4—2.1 (4H, m), 0.5 (4H disappears with $D_2O$).

Example 28

N-[2-(4-(2-Methylaminoethoxy)phenyl)-1-methylethyl]-2-hydroxy-2-(3-chlorophenyl)ethanamine dihydrochloride — (RR,SS) diastereoisomer

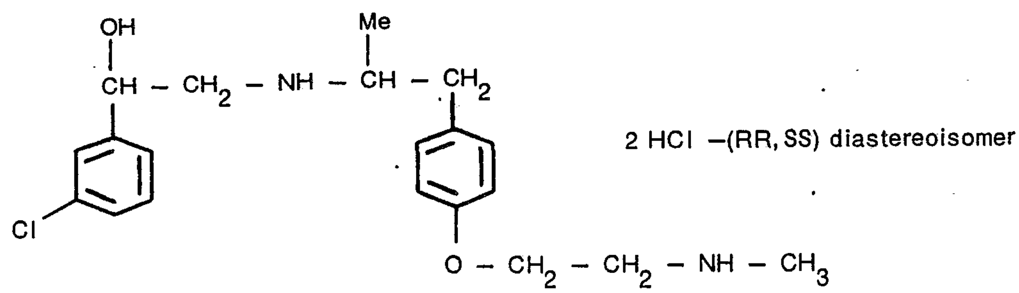

N - [2 - (4 - Carbomethoxymethoxyphenyl) - 1 - methylethyl] - 2 - hydroxy - 2 - (3 - chlorophenyl)-ethanamine hydrobromide ([RR,SS] diastereoisomer of 97% diastereoisomeric purity) was reacted with methylamine by an analogous procedure to that described in Description 18. Subsequent reduction of the amide formed above with borane-methyl sulphide in dry tetrahydrofuran by an analogous procedure to that described in Example 23 gave N - [2 - (4 - (2 - methylaminoethoxy)phenyl) - 1 - methylethyl] - 2 - hydroxy - 2 - (3 - chlorophenyl)ethanamine dihydrochloride as its (RR,SS) diastereoisomer of 95% diastereoisomeric purity, mp 232—4°C (MeOH—EtOAc), of analytical purity.

$^1$H nmr

Identical with that of Example 22.

Example 29

N-[2-(3-(2-Methylaminoethoxy)phenyl)-1-methylethyl]-2-hydroxy-2-(3-chlorophenyl)ethanamine dihydrochloride

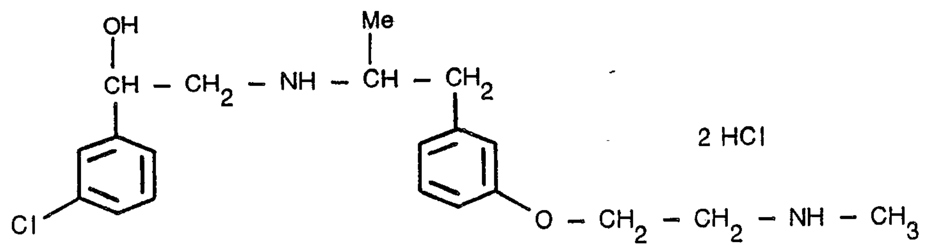

N - [2 - (3 - Carbomethoxymethoxyphenyl) - 1 - methylethyl] - 2 - hydroxy - 2 - (3 - chlorophenyl)-ethanamine was reacted with methylamine by an analogous procedure to that described in Description 18. Subsequent reduction of the amide formed above with borane-methyl sulphide in dry tetrahydrofuran by an analogous procedure to that described in Example 23 gave N - [2 - (3 - (2 - methylaminoethoxy)-phenyl) - 1 - methylethyl] - 2 - hydroxy - 2 - (3 - chlorophenyl)ethanamine dihydrochloride, mp 202—5°C (MeOH—EtOAc) as a 75:25 mixture of diastereoisomers.

$^1$H nmr τ(DMSO-$d_6$)

8.9 (3H, d), 7.4 (3H, s), 6.3—7.3 (7H, complex), 5.75 (2H, t), 4.9 (1H, m), 3.65 (1H, exchange with $D_2O$), 2.4—3.2 (8H, complex), 0.65 (4H, exchange with $D_2O$).

Example 30

N-[2-(4-(2-(1-Morpholine)ethoxy)phenyl)-1-methylethyl]-2-hydroxy-2-(3-trifluoromethylphenyl)ethanamine

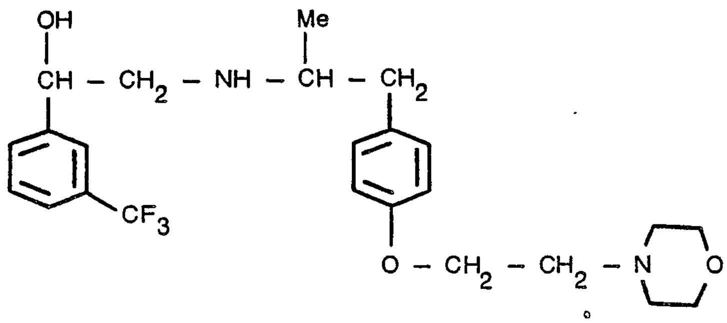

This was prepared in an identical manner to the compound described in Example 5 using 2 - hydroxy - 2 - (3 - trifluoromethylphenyl)ethanamine and 1 - [4 - (2 - (1 - morpholine)ethoxy)phenyl]propan - 2 - one. N - [2 - (4 - (2 - (1-Morpholine)ethoxy)phenyl) - 1 - methylethyl] - 2 - hydroxy - 2 - (3 - trifluoromethylphenyl)ethanamine, mp 96—106°C (ethyl acetate-ether) was isolated as an 18:82 mixture of diastereoisomers.

$^1$H nmr τ(DMSO-$d_6$)

8.8 (3H, d), 7.7—7.3 (11H, complex), 6.8 (2H, disappears with $D_2O$), 6.6—6.4 (4H, complex), 6.0 (2H, t), 5.34 (1H, m), 3.3 (2H, d), 2.9 (2H, d), 2.5—2.34 (4H, m).

Example 31

N-[2-(4-(2-Benzylaminopropoxy)phenyl)-1-methylethyl]-2-hydroxy-2-(3-trifluoromethylphenyl)ethanamine

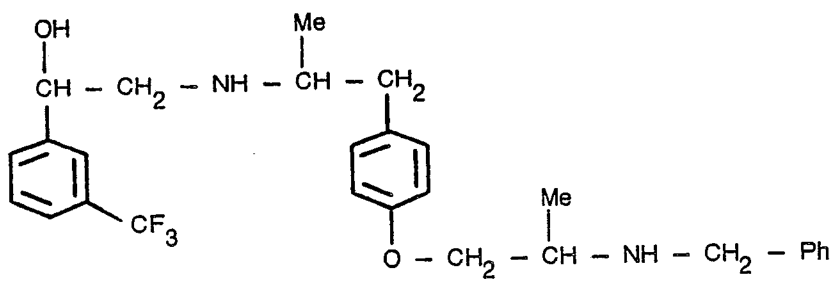

This was prepared in an identical manner to the compound described in Example 5 using 2 - hydroxy - 2 - (3 - trifluoromethylphenyl)ethanamine and 1 - [4 - (2 - benzylaminopropoxy)phenylpropan - 2 - one. N - [2 - (4 - (2 - Benzylaminopropoxy)phenyl) - 1 - methylethyl] - 2 - hydroxy - 2 - (3 - trifluoromethylphenyl)ethanamine, mp 81—92°C (hexane) was isolated as a 94:6 mixture of diastereoisomers.

$^1$H nmr τ($CDCl_3$)

8.9 (3H, d), 8.8 (3H, d), 7.8—6.6 (9H, complex), 6.15 (2H, d, +2H, s), 5.3 (1H, m), 3.2 (2H, d), 2.9 (2H, d), 2.6 (5H, s), 2.6—2.3 (4H, m).

Example 32

N-[2-(4-(2-Aminopropoxy)phenyl)-1-methylethyl]-2-hydroxy-2-(3-trifluoromethylphenyl)ethanamine

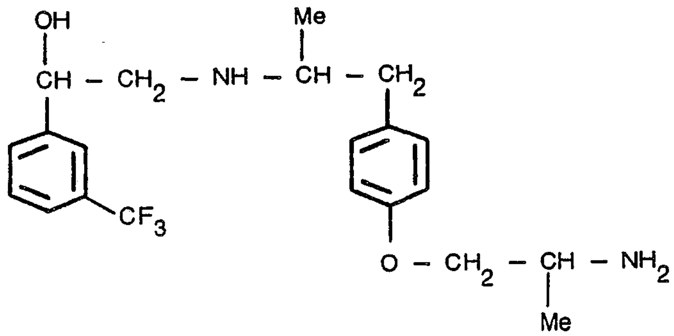

This was prepared from the compound of Example 31 as the dihydrochloride salt using the hydrogenolysis conditions described in Example 19. N - [2 - (4 - (2 - Aminopropoxy)phenyl) - 1 - methylethyl] - 2 - hydroxy - 2 - (3 - trifluoromethylphenyl)ethanamine)phenylethanamine was isolated as an oil.

$^1$H nmr τ($CDCl_3$)

8.85 (3H, d), 8.8 (3H, d), 7.7—6.8 (10H, complex), 6.25 (2H, d), 5.25 (1H, m), 3.2 (2H, d), 2.9 (2H, d), 2.6—2.2 (4H, m).

Example 33

N-[2-(4-(6-Methylaminohexyloxy)phenyl)-1-methylethyl]-2-hydroxy-2-(3-trifluoromethylphenyl)-ethanamine dihydrochloride

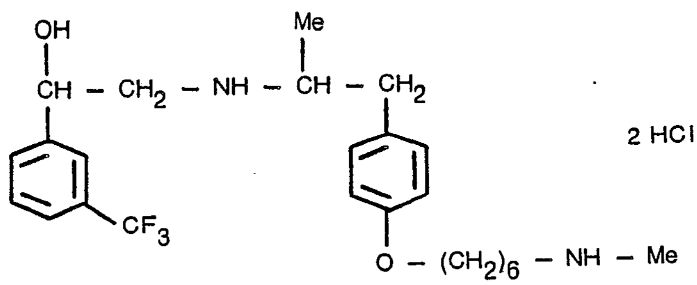

2 HCl

This was prepared in an identical manner to the compound described in Example 5 using 2 - hydroxy - 2 - (3 - trifluoromethylphenyl)ethanamine and 1 - [4 - (6 - methylaminohexyloxy)phenyl]propan - 2 - one. N - [2 - (4 - (6 - Methylaminohexyloxy)phenyl) - 1 - methylethyl] - 2 - hydroxy - 2 - (3 - trifluoromethylphenyl)ethanamine dihydrochloride, mp 165—171°C (methanol-ethylacetate-ether) was isolated as a 50:50 mixture of isomers.

$^1$H nmr τ(DMSO-$d_6$)

8.85 (3H, dd), 8.6 (4H, m), 8.38 (4H, m), 7.55 (3H, s), 7.3 (3H, m), 7.0—6.3 (4H, m), 6.15 (2H, t), 4.75 (1H, m), 3.6 (1H, broad, disappears with $D_2O$), 3.15 (2H, d), 2.85 (2H, d), 2.6—2.1 (4H, m), [0.8 (3H, broad) 0.2 (1H, broad), all disappear with $D_2O$].

Example 34

N-[2-(4-(2-Aminoethoxy)phenyl)ethyl]-2-hydroxy-2-(3-trifluoromethylphenyl)ethanamine

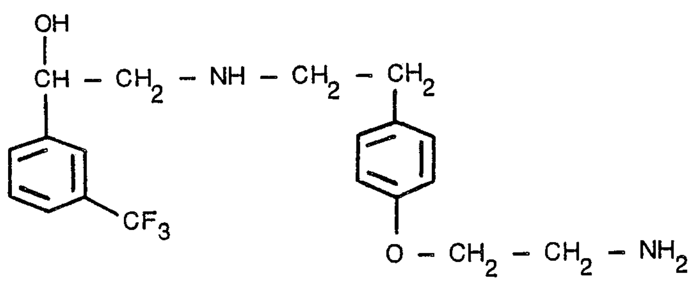

A solution of N - [2 - (4 - (cyanomethoxy)phenyl)ethyl] - 2 - hydroxy - 2 - (3 - trifluoromethylphenyl)ethanamine (1.89 g) in dry tetrahydrofuran was added dropwise under nitrogen to lithium aluminium hydride (0.4 g) in dry tetrahydrofuran. The solution was heated under reflux for 1 h at the end of the addition. Water (0.4 ml), 2M sodium hydroxide (0.4 ml) followed by water (1.2 ml) was added dropwise, the mixture filtered and the filtrate dried (magnesium sulphate). Filtration and evaporation of the solvent gave N - [2 - (4 - (2 - aminoethoxy)phenyl) - ethyl] - 2 - hydroxy - 2 - (3 - trifluoromethylphenyl)-ethanamine, mp 63—67°C (ethyl acetate-ether).

$^1$H nmr τ(DMSO-$d_6$)

7.6—7.2 (6H, complex), 7.2 (2H, t), 7.2—6.7 (4H, broad disappears with $D_2O$), 6.15 (2H, t), 5.25 (1H, m), 3.15 (2H, d), 2.9 (2H, d), 2.5—2.2 (4H, m).

Example 35

N-[3-(4-(2-Dimethylaminoethoxy)phenyl)-1,1-dimethylpropyl]-2-hydroxy-2-phenylethanamine dihydrochloride

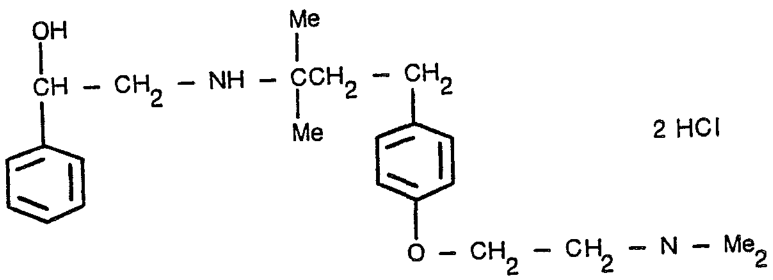

A mixture of 4 - [4 - (2 - dimethylaminoethoxy)phenyl] - 2 - methylbutan - 2 - amine (2.08 g) and phenylglyoxal (1.26 g) was heated under reflux in benzene for 4 h using a Dean and Stark apparatus. The solvent was evaporated, replaced with methanol and sodium borohydride (3 g) added portionwise. The methanol was evaporated, the residue partitioned between ether and water and the ether layer dried (magnesium sulphate). Filtration and evaporation of the solvent gave an oil which was chromatographed on Kieselgel 60. Elution with methanol-chloroform (4:96) gave N - [3 - (4 - (2 - dimethylaminoethoxy)-phenyl) - 1,1 - dimethylpropyl] - 2 - hydroxy - 2 - phenylethanamine dihydrochloride after treatment with ethereal hydrogen chloride, mp 221—223°C (methanol-ethyl acetate).

$^1$H nmr τ(DMSO-$d_6$)

8.6 (6H, s), 8.3—7.9 (2H, m), 7.4 (2H, s), 7.2 (6H, s), 7.0 (2H, m), 6.55 (2H, t), 5.65 (2H, t), 4.9 (1H, m), 3.8 (1H, disappears with $D_2O$), 3.05 (2H, d), 2.8 (2H, d), 2.55 (5H, m), [1.3 (1H), 0.2 (1H), −1.1 (1H) — all disappear with $D_2O$].

Example 36

N-[2-(4-(2-Diethylaminoethoxy)phenyl)-1-methylethyl]-2-hydroxy-2-(3-trifluoromethylphenyl)ethanamine fumarate

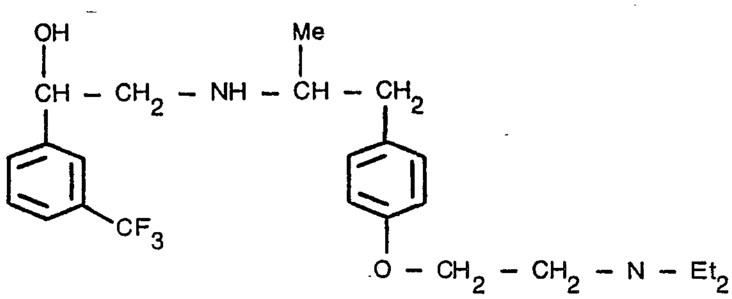

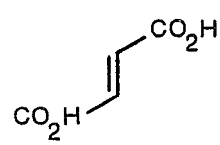

This was prepared in an identical manner to the compound described in Example 5 using 2 - hydroxy - 2 - (3 - trifluoromethylphenyl)ethanamine and 1 - [4 - (2 - diethylaminoethoxy)phenyl]propan - 2 - one. N - [2 - (4 - (2 - Diethylaminoethoxy)phenyl) - 1 - methylethyl] - 2 - hydroxy - 2 - (3 - trifluoromethylphenyl)ethanamine fumarate, mp 136—138°C (methanol-ethylacetate) was isolated as a 52:48 mixture of diastereoisomers.

$^1$H nmr τ(DMSO-$d_6$)

9.0 (6H, t + 3H, d), 7.3 (4H, q), 7.1 (7H, complex), 5.9 (2H, t), 4.95 (1H, m), 3.45 (2H, s), 3.15 (2H, d), 2.85 (2H, d), 2.5—1.8 (4H, m + 4H, disappears with $D_2O$).

Example 37

N-[2-(4-(Cyanomethoxy)phenyl)-ethyl]-2-hydroxy-2-(3-trifluoromethylphenyl)ethanamine

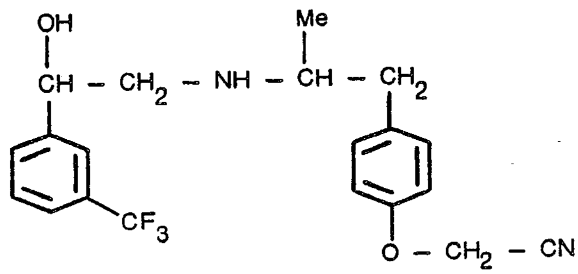

A mixture of 2-hydroxy-2-(3-trifluoromethylphenyl)ethanamine (1.45 g), triethylamine (1.5 ml) and 2-[4-(cyanomethoxy)phenyl]ethanol, 4-toluene sulphonate (2.0 g) was stirred in dimethyl sulphoxide for 5 days at ambient temperature. The mixture was poured into water and extracted with ethyl acetate. The combined ethyl acetate layers were washed with water (×2) and dried (magnesium sulphate). Evaporation of the solvent gave an oil which was chromatographed on Kieselgel 60. Elution with methanol-chloroform (2:98) gave the title compound as an oil.

$^1$H nmr τ($CDCl_3$)

7.4 (2H, m), 7.2 (6H, m), 5.25 (2H, + 1 Hm), 3.15 (2H, d), 2.8 (2H, d), 2.6—2.3 (4H, m).

Example 38

N-[2-(4-Cyanomethoxy)phenyl)-1-methylethyl]-2-hydroxy-2-(3-chlorophenyl)ethanamine

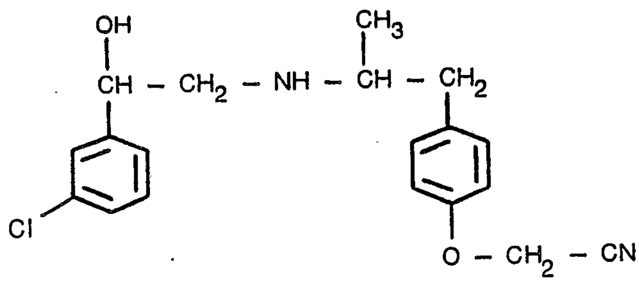

This was prepared in an identical manner to the compound described in Example 1 using 2 - hydroxy - 2 - (3 - chlorophenyl)ethanamine and 1 - [(4 - cyanomethoxy)phenyl]propan - 2 - one. N - [2 - (4 - cyanomethoxy)phenyl) - 1 - methylethyl] - 2 - hydroxy - 2 - (3 - chlorophenyl)ethanamine, mp 74—79°C (ether hexane) was isolated as a 31:69 mixture of diastereoisomers.

$^1$H nmr τ($CDCl_3$)

8.9 (3H, d), 6.9—7.6 (5H, complex), 6.45 (2H, broad singlet, exchanges with $D_2O$), 5.1—5.3 (3H, complex), 2.5—3.3 (8H, complex).

Description 1

1-[4-(2-Hydroxyethoxy)phenyl]propan-2-one ethylene ketal

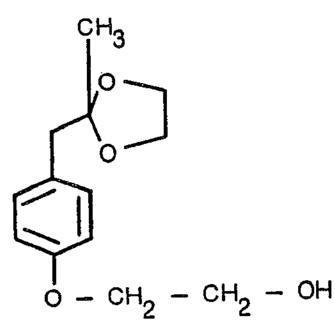

A mixture of 1-(4-carbomethoxymethoxyphenyl)propan-2-one ethylene ketal (5.0 g) in methanol (70 ml) was treated portionwise with sodium borohydride (5.0 g) with stirring over 30 minutes then stirred for a further 30 minutes. Water (150 ml) was added and the mixture extracted with dichloromethane, dried (magnesium sulphate) and evaporated to dryness to give the title compound.

$^1$H nmr $\tau(CDCl_3)$

8.7 (3H, s), 7.5 (1H), 7.15 (2H, s), 5.8—6.55 (8H, complex), 3.15 (2H, d), 2.8 (2H, d).

Description 2

1-[4-(2-Hydroxyethoxy)phenyl]propan-2-one ethylene ketal p-toluene sulphonic ester

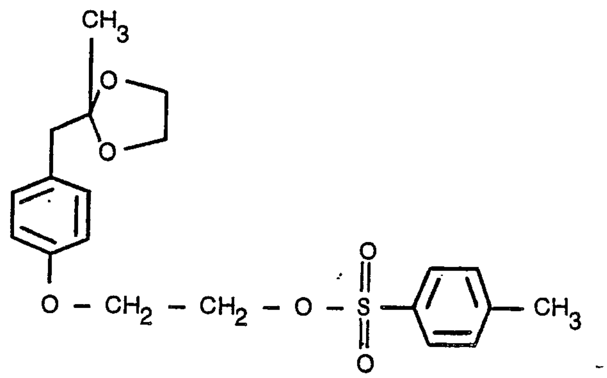

p-Toluenesulphonyl chloride (3.8 g) was added portionwise to a stirred solution of 1-[4-(2-hydroxyethoxy)phenyl]propan-2-one ethylene ketal (4.5 g) in pyridine (100 ml) at 0°C, stirred for 2 hours at 0°C, one hour at room temperature, poured into iced water (250 ml) and extracted with diethyl ether. The extracts were washed with dilute hydrochloric acid, water, dried (magnesium sulphate), filtered and evaporated to dryness to give the title compound, M.P. 68—70°C.

$^1$H nmr $\tau(CDCl_3)$

8.85 (3H, s), 7.57 (3H, s), 7.25 (2H, s), 6.22 (4H, m), 5.79 (4H, dd), 3.33 (2H, d), 2.91 (2H, d), 2.57 (2H, d), 2.20 (2H, d).

Description 3

1-[4-(2-Methylaminoethoxy)phenyl]propan-2-one

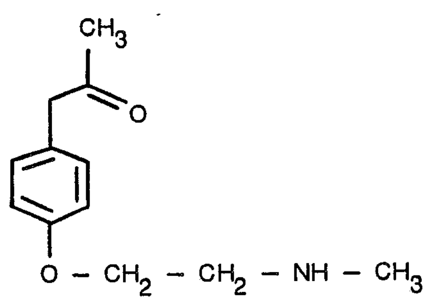

Gaseous methylamine was slowly added to a stirred solution of 1-[4-(2-hydroxyethoxy)phenyl]propan-2-one ethylene ketal p-toluenesulphonate ester (4.0 g) in dimethyl sulphoxide (100 ml) over 2 hours at room temperature. After stirring for a further 2 hours, the mixture was poured into water (800 ml), extracted with the diethyl ether, washed with water, dried (magnesium sulphate) and evaporated to give the title compound as its ethylene ketal.

$^1$H nmr $\tau(CDCl_3)$

8.7 (3H, s), 8.3 (1H, s), 7.5 (3H, s), 7.15 (2H, s), 7.0 (2H, m), 5.8—6.35 (6H, m), 3.2 (2H, d), 2.8 (2H, d).

Deketalisation to the title compound was effected in the usual manner with methanol-2*M* hydrochloric acid at room temperature.

Description 4

1-[4-(2-Dimethylaminoethoxy)phenyl]propan-2-one

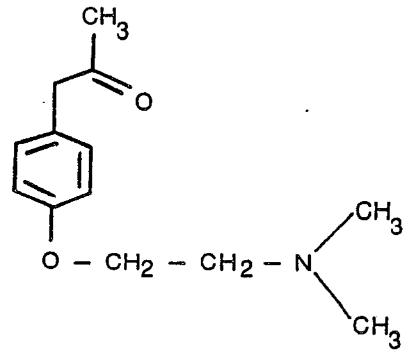

1-[4-(2-Dimethylaminoethoxy)phenyl]propan-2-one ethylene ketal was prepared from 1-[4-(2-hydroxyethoxy)phenyl]propan-2-one ethylene ketal p-toluene sulphonic ester by an analogous procedure to that described in Description 3.

$^1$H nmr τ($CDCl_3$)

8.7 (3H, s), 7.65 (6H, s), 7.3 (2H, m), 7.15 (2H, s), 5.8—6.35 (6H, m), 3.14 (2H, d), 2.8 (2H, d).

Deketalisation was effected with methanol-2*M* hydrochloric acid at room temperature in the usual manner.

Description 5

1-[4-(2-Methylsulphonylaminoethoxy)phenyl]propan-2-one

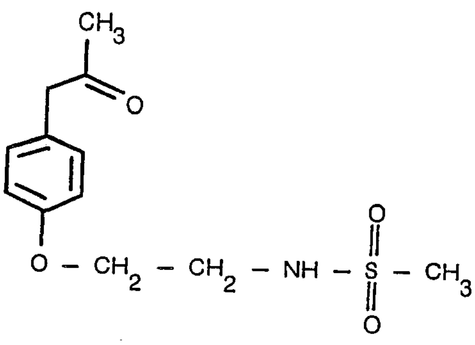

A mixture of 1-(4-hydroxyphenyl)propan-2-one ethylene ketal (3.88 g), N-(2-bromoethyl)methysulphonamide (4.44 g) and sodium hydroxide (1.5 g) in water (30 ml) was heated under reflux for 90 minutes, cooled, diluted with water and extracted with ethylacetate. The extracts were dried (magnesium sulphate) and evaporated to give the title compound as its ethylene ketal. Deketalisation in methanol-2*M* hydrochloric acid was effected in the usual manner.

Description 6

1-(3-Fluoro-4-hydroxyphenyl) propan-2-one

i) *2-Chloro-1-(3-fluoro-4-hydroxyphenyl) propan-1-one*

2-Chloropropionyl chloride (55 g) was added, dropwise, under nitrogen gas, to a stirred slurry of aluminium chloride (116 g) in 1,2-dichloroethane (200 ml). The mixture was stirred at ambient temperatures for 15 minutes. 2-Fluoroanisole (50 g) was added dropwise to the stirred mixture; the reaction mixture was stirred at ambient temperatures for 16 hours and poured onto an ice-water mixture. The organic layer was separated and the aqueous layer was extracted with 1,2-dichloroethane. The combined organic layers were washed with water, dried (magnesium sulphate) and concentrated to give 2-Chloro-1-(3-fluoro-4-hydroxyphenyl) propan-1-one as an oil which was used in the next stage without purification.

ii) *1-(3-Fluoro-4-hydroxyphenyl) propane-1,2-diol*

Potassium acetate (40 g) then glacial acetic acid (20 ml) and then sodium iodide (10 g) were added to a solution of 2-chloro-1-(3-fluoro-4-hydroxyphenyl)propan-1-one (80 g) in acetone (350 ml). The mixture was stirred and heated under reflux for 4 hours, cooled, and treated with water (250 ml). The acetone was evaporated and the aqueous residue was extracted into dichloromethane. The organic phase was washed

with sodium bicarbonate solution, dried (magnesium sulphate) and concentrated by removal of the solvent under reduced pressure. The residual oil was dissolved in ethanol (400 ml), cooled in ice and treated with excess sodium borohydride (15 g).

The solution was stirred for 2 hours at ambient temperatures, the ethanol evaporated and the residue partitioned between dilute hydrochloric acid and ethyl acetate. The ethyl acetate layer was dried (magnesium sulphate) and evaporated to give 1-(3-fluoro-4-hydroxyphenyl) propane-1,2-diol as an oil which was used in the next stage without purification.

iii) *1-(3-Fluoro-4-hydroxyphenyl) propan-2-one*

A mixture of 1-(3-fluoro-4-hydroxyphenyl) propane-1,2-diol (38 g) and p-toluene sulphonic acid (1 g) in benzene (150 ml) was heated under reflux with azeotropic removal of water using a Dean and Stark head for 2 hours. The solvent was removed by evaporation and the residue partitioned between water and dichloromethane. The organic layer was washed with sodium bicarbonate solution, dried (magnesium sulphate) and concentrated to give an oil which was purified by column chromatography on silica gel. 1-(3-Fluoro-4-hydroxyphenyl) propan-2-one was isolated as a low-melting crystalline solid.

τ($CDCl_3$)

7.75 (3H, S); 6.3 (2H, S); 6.15 (1H, S); 2.8—3.2 (3H, m)

Description 7

1-[3-Fluoro-4-carbomethoxymethoxyphenyl]propan-2-one

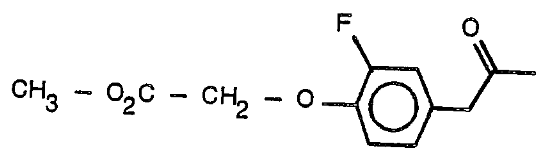

A mixture of 1-(3-fluoro-4-hydroxyphenyl)-propan-2-one (10.0 g), methyl bromoacetate (10.0 g), potassium carbonate (10.0 g) in acetone (200 ml) was heated under reflux, with stirring, for three hours, cooled, filtered and evaporated to dryness to give 1-[3-Fluoro-4-carbomethoxymethoxyphenyl] propan-2-one which was used without further purification.

$^1$H nmr τ($CDCl_3$)

7.8 (3H, S), 6.35 (2H, S), 6.15 (3H, S), 5.3 (2H, S), 2.8—3.25 (3H, complex)

The above ketone was converted to its ethylene ketal with ethylene glycol in benzene under reflux using a catalytic quantity of p-toluenesulphonic acid, in the usual manner.

$^1$H nmr τ($CDCl_3$)

8.7 (3H, S), 7.15 (2H, S), 6.0—6.4 (7H, S on M), 5.3 (2H, S), 2.8—3.3 (3H, complex)

Description 8

1-[3-Fluoro-4-(2-hydroxyethoxy)phenyl] propan-2-one ethylene ketal

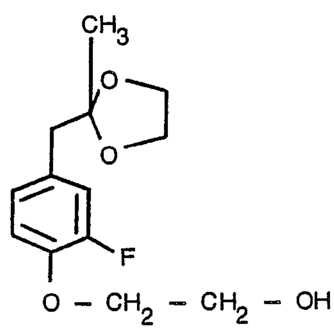

1-[3-Fluoro-4-(2-hydroxyethoxy)phenyl] propan-2-one ethylene ketal was prepared using 1-(3-fluoro-4-carbomethoxymethoxyphenyl) propan-2-one ethylene ketal by an analogous procedure to that described in Description 1.

$^1$H nmr τ($CDCl_3$)

8.70 (3H, s), 7.15 (3H, broad s), 5.7—6.35 (8H, complex), 2.8—3.2 (3H, m).

Description 9

1-[3-Fluoro-4-(2-hydroxyethoxy)phenyl] propan-2-one ethylene ketal p-toluene sulphonic acid ester

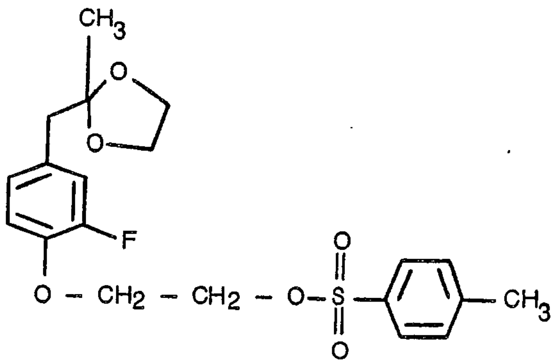

1-[3-Fluoro-4-(2-hydroxyethoxy)phenyl] propan-2-one ethylene ketal p-toluene sulphonic acid ester, m.p. 79—80° (ethanol) was prepared from p-toluenesulphonyl chloride (4.2 g) and 1-[3-fluoro-4-(2-hydroxyethoxy)phenyl] propan-2-one ethylene ketal (5.0 g) by an analogous procedure to that described in Description 2.

$^1$H nmr τ($CDCl_3$)

8.85 (3H, s) 7.65 (3H, s), 7.3 (2H, s), 6.2—6.5 (4H, m), 5.7—6.1 (4H, m), 3.0—3.5 (3H, complex), 2.7 (2H, d), 2.2 (2H, d).

Description 10

1-[3-Fluoro-4-(2-methylaminoethoxy)phenyl] propan-2-one

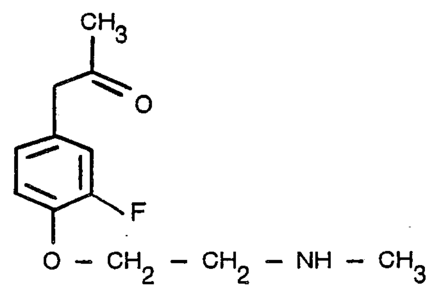

1-[3-Fluoro-4-(2-methylaminoethoxy)phenyl] propan-2-one was prepared from 1-[3-fluoro-4-(2-hydroxyethoxy)phenyl] propan-2-one ethylene ketal p-toluenesulphonate ester by an analogous procedure to that described in Description 3. The oil formed was used without further purification.

Description 11

1-[4-(2-Benzylaminoethoxy)phenyl]propan-2-one

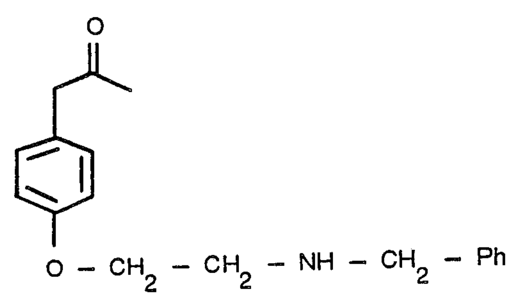

A mixture of benzylamine (4.91 g) and 1-[4-(2-hydroxyethoxy)phenyl]propan-2-one ethylene ketal p-toluene sulphonate ester (17.24 g) was heated at 60°C in dimethyl sulphoxide for 6 h. The reaction mixture was poured into ice-water, the solution extracted with ether, the ether layers washed with water and dried.

Evaporation of the solvent gave the title compound as its ethylene ketal (13.6 g). Deketalisation of 5 g of this material with methanol-2M-hydrochloric acid under normal conditions gave 1-[4-(2-benzylaminoethoxy)-phenyl] propan-2-one, (4.6 g).

$^1$H nmr τ($CDCl_3$)

7.9 (3H, s), 7.0 (2H, t), 6.4 (2H, s), 6.2 (2H, s), 5.95 (2H, t + 1H), 3.2 (2H, d), 2.9 (2H, d), 2.7 (5H, s).

Description 12

1-[4-(2-(1-Piperidine)ethoxy)phenyl]propan-2-one

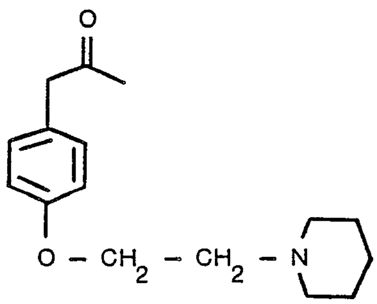

This material was prepared in an identical manner to that described in Description 3 using piperidine instead of methylamine.

$^1$H nmr τ($CDCl_3$)

8.1—8.7 (6H, m), 7.95 (3H, s), 7.4—7.7 (4H, m), 7.3 (2H, t), 6.45 (2H, s), 6.0 (2H, t), 3.2 (2H, d), 2.9 (2H, d).

Description 13

1-(4-(2-(1-Homopiperidine)ethoxy)phenyl]propan-2-one

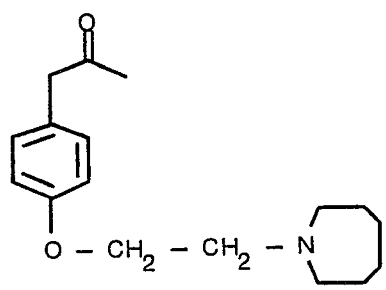

This material was prepared in an identical manner to that described in Description 3 using homopiperidine instead of methylamine.

$^1$H nmr τ($CDCl_3$)

8.1—8.5 (8H, m), 7.9 (3H, s), 6.8—7.4 (6H, m), 6.4 (2H, s), 6.0 (2H, t), 3.2 (2H, d), 2.9 (2H, d).

Description 14

1-[4-(2-Ethoxycarbonylaminoethoxy)phenyl]propan-2-one

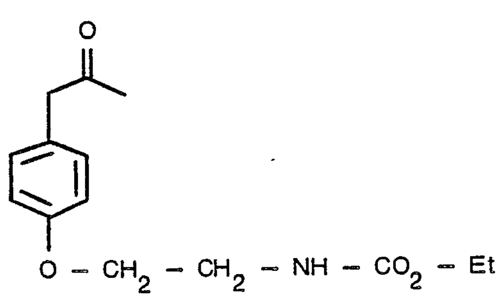

Ethyl chloroformate (4.4 g) was added to a solution of 1-[4-(2-aminoethoxy)phenyl]propan-2-one, ethylene ketal (8.0 g) in pyridine at 0°C and the solution left at this temperature for 1 h. Water was added, followed by 2M hydrochloric acid until the solution was acidic and then extracted with dichloromethane.

The organic layers were washed with 2M hydrochloric acid, dried, and evaporated to give the title compound as the ethylene ketal (7.01 g). This was deketalised with methanol-2M hydrochloric acid at room temperature to give the title compound (4.13 g).

$^1$H nmr $\tau$($CDCl_3$)

8.85 (3H, t), 7.9 (3H, s), 6.4 (2H, s), 6.4 (2H, t), 5.7—6.2 (4H, m), 4.7 (1H, broad), 3.2 (2H, d), 2.9 (2H, d).

Description 15

1-[4-(2-Aminoethoxy)phenyl]propan-2-one, ethylene ketal

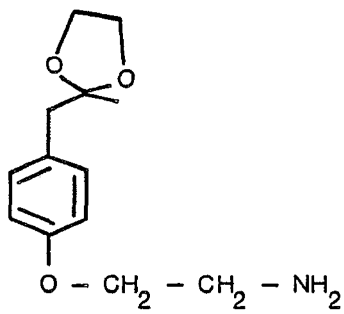

Anhydrous ammonia was passed through a solution of 1-[4-(2-hydroxyethoxy)phenyl]propan-2-one, ethylene ketal, p-toluene sulphonic ester (1.86 g) in dimethylsulphoxide (30 ml) for 2 h at room temperature. The mixture was stirred for 15 h at 50°C, cooled and water (50 ml) was added. The solution was extracted with ether, the organic extracts dried and evaporated to give the title compound.

$^1$H nmr $\tau$($CDCl_3$)

8.8 (3H, s), 8.3 (2H, broad, disappears with $D_2O$), 7.2 (2H, s), 6.95 (2H, t), 5.7—6.3 (6H, m), 3.3 (2H, d), 2.9 (2H, d).

Description 16

1-[4-(2-Methylaminocarbonylaminoethoxy)phenyl]propan-2-one

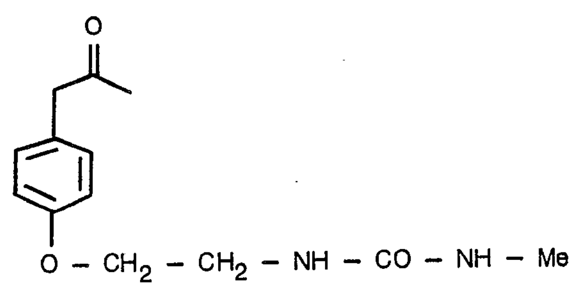

A solution of 1-[4-(2-Aminoethoxy)phenyl]propan-2-one, ethylene ketal (3.5 g) was heated under reflux in toluene (20 ml) containing phosgene for 2 h. The solvent was evaporated, benzene added and evaporated. The residue was taken up in dry tetrahydrofuran and methylamine passed through the solution for 0.5 h. The solvent was evaporated, the residue taken up in chloroform and washed with 2M hydrochloric acid. The organic layer was dried and evaporated to give the title compound as its ethylene ketal (3.35 g). Deketalisation was accomplished with methanol/2M hydrochloric acid at room temperature.

$^1$H nmr $\tau$($CDCl_3$)

7.8 (3H, s), 7.25 (3H, d), 6.35 (2H, s + 2H, m), 6.0 (2H, t), 4.75 (1H, m), 4.5 (1H, m), 3.2 (2H, d), 2.85 (2H, d).

Description 17

1-[4-(2-Acetylaminoethoxy)phenyl]propan-2-one

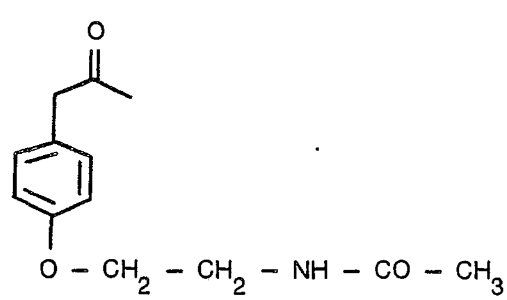

This material was prepared in a similar manner to that described in Description 14, using acetyl chloride instead of ethyl chloroformate.

$^1H$ nmr $\tau(CDCl_3)$

8.05 (3H, s), 7.9 (3H, s), 6.45 (2H, s), 5.8—6.4 (4H, complex), 3.25 (2H, d), 2.95 (2H, d).

Description 18

N-[2-(4-(2-Methylaminocarbonylmethoxy)phenyl-1-methylethyl]-2-hydroxy-2-(3-trifluoromethylphenyl)-ethanamine

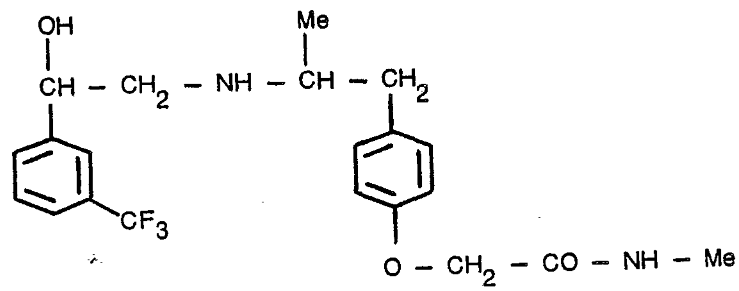

Aqueous methylamine (80 ml of a 25—30% solution) was added in 4 portions to a solution of N - [2 - (4 - (2 - methoxycarbonylmethoxy)phenyl) - 1 - methylethyl] - 2 - hydroxy - 2 - (3 - trifluoromethylphenyl)ethanamine in methanol at reflux. The solution was heated under reflux for 2.5 h, cooled to room temperature, a further 50 ml of methylamine solution added and the solution was allowed to stand at ambient temperature 16 h. The solution was evaporated to half-volume, brine was added and the solution was extracted with dichloromethane. The extracts were washed with brine, dried and evaporated to give the title compound, mp 75—80°C.

$^1H$ nmr $\tau(CDCl_3)$

8.95 (3H, d), 6.9—7.7 (10H complex), 5.6 (2H, s), 5.35 (1H, m), 3.35 (1H, broad, disappears with $D_2O$), 3.2 (2H, d), 2.95 (2H, d), 2.45 (4H, complex).

Passage of hydrogen bromide through a solution of the title compound in ethyl acetate-ether for 5 min at 0°C precipitated a solid which after two recrystallizations from methanol-ethyl acetate gave the (RR, SS) diastereoisomer, as the hydrobromide salt, mp 199—200°C.

The original mother liquor was treated with saturated sodium bicarbonate solution, dried, and evaporated. Trituration with hexane followed by two recrystallizations of the resulting solid from ether-hexane gave the (RS,SR) diastereoisomer, mp 83—85°C.

Description 19

1-[4-(2-(1-Pyrrolidine)ethoxy)phenyl]propan-2-one

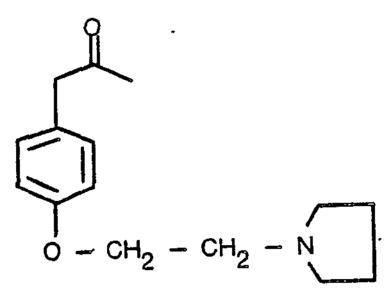

This material was prepared in an identical manner to that described in Description 3 using pyrrolidine instead of methylamine.

$^1H$ nmr $\tau(CDCl_3)$

8.2 (4H, m), 7.85 (3H, s), 7.5 (4H, m), 7.15 (2H, t), 6.35 (2H, s), 5.9 (2H, t), 3.2 (2H, d), 2.9 (2H, d).

Description 20

N-(2-(4-(2-Ethylaminocarbonylmethoxy)phenyl)-1-methylethyl]-2-hydroxy-2-(3-trifluoromethylphenyl)-ethanamine

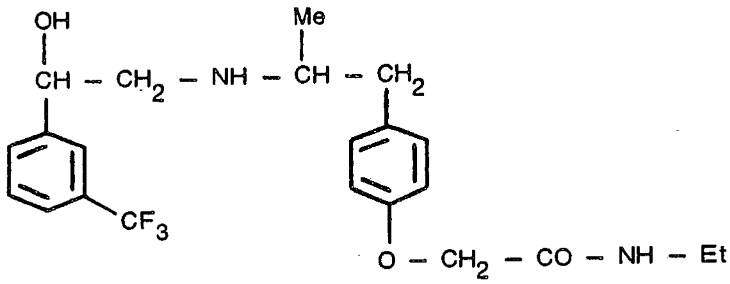

This material was prepared in an identical manner to that described in Example 5 using 2-hydroxy-2-(3-trifluoromethylphenyl)ethanamine and 1-(4-(ethylaminocarbonylmethoxy)phenyl)propan-2-one.

$^1$*H nmr* τ (DMSO-$d_6$)

9.1 (3H, d), 8.95 (3H, t), 8.0 (1H, broad), 7.7—7.0 (5H, m), 6.85 (2H, m), 5.55 (2H, s), 5.3 (1H, t), 4.5 (1H, broad), 3.15 (2H, d), 2.9 (2H, d), 2.5—2.2 (4H, m), 1.9 (1H, m).

Description 21

N-[2-(4-Carbomethoxymethoxyphenyl)-1-methylethyl]-2-hydroxy-2-(3-chlorophenyl)ethanamine hydrobromide-(RR,SS) diastereoisomer

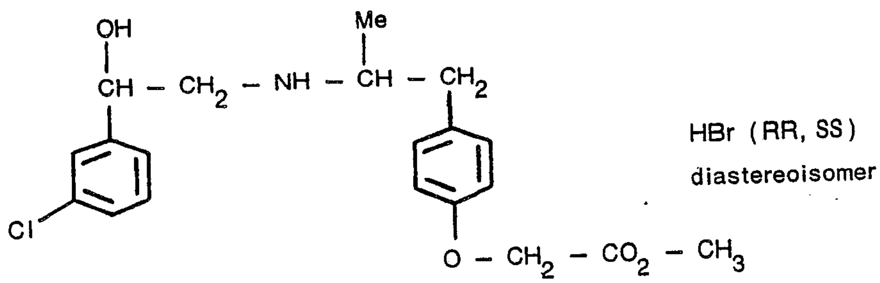

N - [2 - (4 - Carbomethoxymethoxyphenyl) - 1 - methylethyl] - 2 - hydroxy - 2 - (3 - chlorophenyl)-ethanamine (4.0 g; 58:42 mixture of diastereoisomers) was suspended in diethylether (100 ml) and sufficient methanol added to obtain solution. Hydrogen bromide was passed through the solution for 15 minutes at 0—5°C. The top ethereal layer was decanted from the lower oily layer and ethyl acetate (50 ml) added. After stirring for 30 minutes at 0°C the solid formed was removed by filtration to give the title compound, mp 137—8°C, of analytical purity and 96% diastereoisomeric purity.

$^1$*H nmr* τ (DMSO-$d_6$)

8.9 (3H, s), 6.5—7.4 (5H, complex), 6.3 (3H, s), 5.25 (2H, s), 4.95 (1H, q), 3.75 (1H, exchanges with $D_2O$), 3.1 (2H, d), 2.8 (2H, d), 2.5 (4H, m), 1.2 (2H, exchanges with $D_2O$).

Description 22

N-[2-(3-Carbomethoxymethoxyphenyl)-1-methylethyl]-2-hydroxy-2-(3-chlorophenyl)ethanamine

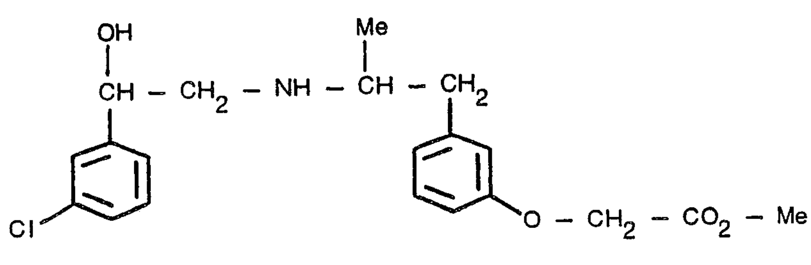

The title compound, mp 62—8°C was prepared, as a mixture of diastereoisomers of analytical purity, from 2-hydroxy-2-(3-chlorophenyl)ethanamine (3.71 g) and 1-(3-carbomethoxymethoxyphenyl) propan-2-one (4.80 g) by an analogous procedure to that described in Example 1.

$^1$*H nmr* τ ($CDCl_3$)

8.95 (3H, d), 6.95—7.7 (7H, complex; 2H exchange with $D_2O$), 6.25 (3H, s), 5.3—5.6 (3H, s on m), 3.1—3.4 (3H, complex) 2.55—2.95 (5H, complex).

Description 23

1-[4-(2-(1-Morpholine)ethoxy)phenyl]propan-2-one

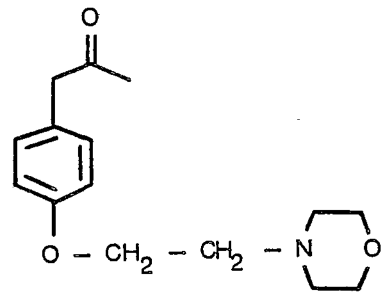

This material was prepared in an identical manner to that described in Description 3 using morpholine instead of methylamine.

$^1H$ *nmr* τ($CDCl_3$)

7.9 (3H, s), 7.65—7.2 (4H, m), 7.2 (2H, t), 6.4 (2H, s), 6.3 (2H, t), 6.1—5.7 (4H, t), 3.15 (2H, d), 2.8 (2H, d).

Description 24

1-[4-(2-Benzylaminopropoxy)phenyl]propan-2-one

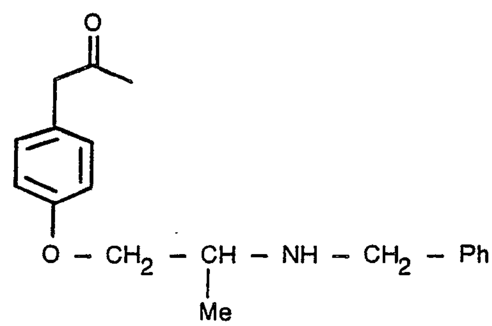

A mixture of 4-hydroxyphenylpropanone, ethylene ketal (7.0 g), chloroacetone (3.32 g) and potassium carbonate (16.1 g) was heated under reflux for 16h in acetone containing a trace of potassium iodide. The solution was evaporated, the residue partitioned between ether and water, the layers separated and the ether layer dried (magnesium sulphate). Filtration and removal of the solvent gave an oil (8.4 g). This was not purified further but heated under reflux for 4h with benzylamine (3.6 g) in benzene under Dean and Stark conditions. The solvent was evaporated, the residue dissolved in methanol and sodium borohydride (2.6 g) added portionwise. The methanol was evaporated, the residue partitioned between ether and water, the layers separated and the ether layer dried. Removal of the solvent gave an oil which was dissolved in acetone-2M hydrochloric acid and the solution left at room-temperature for 48h. The acetone was evaporated, 2M sodium hydroxide solution added and the mixture extracted with ether, and the ether layers dried. Removal of the solvent gave the title compound as an oil (7.4 g).

$^1H$ *nmr* τ ($CDCl_3$)

8.8 (3H, d), 7.9 (3H, s), 7.4 (1H, broad), 6.9 (1H, m), 6.4 (2H, s), 6.2 (1H, s + 2H, d), 3.2 (2H, d), 2.9 (2H, d), 2.7 (5H, m).

Description 25

1-[(4-Cyanomethoxy)phenyl]propan-2-one

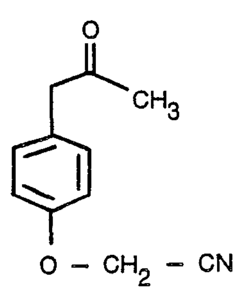

Chloroacetonitrile (4.0 g) was added to a solution of (4-hydroxyphenyl)propan-2-one, ethylene ketal (9.7 g) in acetone (100 ml) containing potassium iodide (5.0 g) and potassium carbonate (7.0 g). The mixture was boiled under reflux with stirring for 8 hours. The mixture was cooled, filtered and the solvent evaporated. The residue was dissolved in ether, washed with dilute sodium hydroxide solution, brine, dried (magnesium sulphate) filtered and evaporated. The residual oil was taken up in methanol — 2M hydrochloric acid and allowed to stand at ambient temperature for 2 hours. Water was added, the solution extracted with ether and the combined organic layers dried (magnesium sulphate). Evaporation of the solvent gave 1-[(4-cyanomethoxy)phenyl]propan-2-one.

$^1H$ *nmr* τ ($CDCl_3$)

7.85 (3H, s); 6.35 (2H, s); 5.30 (2H, s); 3.10 (2H, d); 2.80 (2H, d).

Description 26

2-[4-(Cyanomethoxy)phenyl]ethanol,4-toluene sulphonate

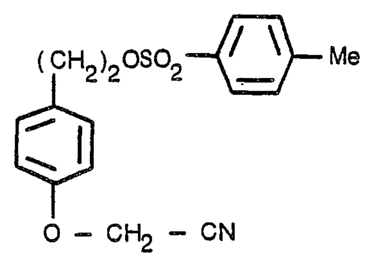

A mixture of 4-hydroxyphenethylalcohol (5.0 g), chloroacetonitrile (2.7 g) and potassium carbonate (10.0 g) was heated under reflux for 24h in acetone containing a trace of potassium iodide. The mixture was filtered, the acetone evaporated and the residue partitioned between chloroform and water. The chloroform layer was washed with 2M sodium hydroxide and dried (magnesium sulphate). Filtration and evaporation of the solvent gave 2 - [4 - (cyanomethoxy)phenyl]ethanbol (7.3 g). This was dissolved in dry pyridine, cooled to 0°C and a solution of 4-toluene sulphonyl chloride added dropwise. The solution was allowed to stand at ambient temperature for 16h then poured into water and extracted with ether. The ether layers were combined, washed with water, 2M hydrochloric acid, water and dried (magnesium sulphate). Filtration and evaporation of the solvent gave the title compound as an oil (8.24 g).

$^1H$ *nmr* τ ($CDCl_3$)

7.5 (3H, s), 7.1 (2H, t), 5.75 (2H, t), 5.3 (2H, s), 3.1 (2H, d), 2.8 (2H, d), 2.65 (2H, d), 2.28 (2H, d).

Description 27

4-[4-(2-Dimethylaminoethoxy)phenyl]-2-methylbutan-2-amine

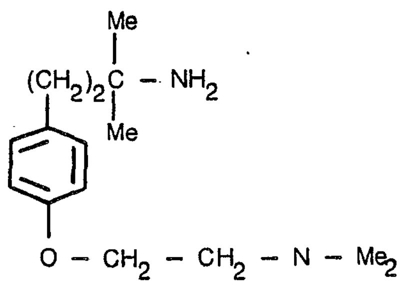

A mixture of 2 - methyl - 4 - (4 - hydroxphenyl)butan - 2 - ol (18.0 g), 2-dimethylaminoethylchloride hydrochloride (14.4 g) and potassium carbonate (54 g) was stirred and heated under reflux for 4 days in acetone containing potassium iodide (16.2 g). The mixture was filtered, the acetone evaporated and the residue was taken up into chloroform. This was extracted with 2M hydrochloric acid and the chloroform discarded. The acid layer was made basic with 2M sodium hydroxide, extracted with chloroform and the organic layers dried. Filtration and evaporation of the solvent gave 4 - ((4 - dimethylaminoethoxy)-phenyl) - 2 - methyl - butan - 2 - ol as a clear oil (4.01 g).

Sodium cyanide (0.6 g) was added to glacial acetic acid (4 ml) kept at *ca* 20°C. To this mixture was added dropwise a cooled solution of concentrated sulphuric acid (2 ml) in glacial acetic acid (2 ml), the temperature being kept to 20°C with an ice bath. The ice-bath was removed and a solution of the above alcohol (3.5 g) in glacial acetic acid was added dropwise. The reaction mixture was allowed to attain ambient temperature and then allowed to stand at ambient temperature for 16h. Nitrogen was bubbled

through the solution for 0.5h, the mixture poured into water, neutralized with solid sodium carbonate, extracted with ethyl acetate, dried and evaporated to give the title compound at its N-formyl derivative (2.8 g).

This was heated under reflux in sodium hydroxide solution (20% w/w) for 24h, the solution cooled, extracted with chloroform, dried and evaporated to give the title compound as an oil (2.08 g).

$^1H$ *nmr* $\tau$ ($CDCl_3$)

8.9 (6H, s), 8.45 (2H, m), 8.3 (2H, m), 7.7 (6H, s), 7.55 (2H, broad), 7.35 (2H, t), 6.0 (2H, t), 3.2 (2H, d), 2.9 (2H, d).

Description 28

1-[4-(2-Diethylaminoethoxy)phenyl]propan-2-one

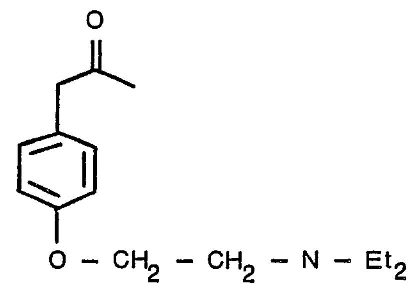

This was prepared in an identical manner to the compound described in Description 3 using diethylamine instead of methylamine.

$^1H$ *nmr* $\tau$ ($CDCl_3$)

8.5 (6H, t), 7.9 (3H, s), 7.5 (4H, q), 7.2 (2H, t), 6.4 (2H, s), 6.0 (2H, t), 3.1 (2H, d), 2.9 (2H, d).

Demonstration of Effectiveness of Compounds

(I) *Anti-hyperglycaemic activity*

Female CFLP mice, each weighing approximately 25 g, were fasted for 24 hours prior to the study. The compounds under study were administered orally as an aqueous solution to each of 6 mice. 6 mice were given water as a control. 30 minutes later a blood sample (20 µl) was obtained from the tail for the analysis of blood glucose. Immediately after taking this blood sample, glucose (1 g/kg body weight) was administered subcutaneously to each mouse. Blood samples were then obtained from each mouse at 30 minute intervals for 120 minutes.

Compounds that produced a significant ($P<0.05$) reduction of blood glucose, compared with control mice given water, at any time interval, were considered active. The area under the blood glucose curve over the 2 hour period after the administration of the glucose was calculated for each compound and compared with the value for control animals.

| Compound of Example No. | Dose ($\mu$ moles /kg po) | Reduction in area under Blood glucose curve (%) |
|---|---|---|
| Control | – | 0 |
| 1 | 1 | 33 |
| 2 | 5 | 49 |
| 3 | 1 | 56 |
| 4 | 2.5 | 18 |
| 5 | 1 | 38 |
| 6 | 50 | 36.5 |
| 7 | 1 | 33 |
| 8 | 1 | 51 |
| 9 | 1 | 39 |
| 10 | 1 | 30 |
| 11 | 1 | 64 |
| 13 | 1 | 62 |
| 14 | 1 | 68 |
| 15 | 1 | 17 |
| 16 | 1 | 68 |
| 18 | 2.5 | 50 |
| 19 | 1 | 46 |
| 20 | 2.5 | 36 |
| 21 | 2.5 | 44 |
| 22 | 1 | 43 |
| 23 | 2.5 | 20 |
| 24 | 1 | 47 |
| 28 | 0.5 | 42 |
| 29 | 12.5 | 48 |
| 31 | 50 | 45 |
| 35 | 250 | 18 |
| 36 | 50 | 56 |
| 38 | 0.5 | 63 |

(II) *Effect on energy expenditure*

The effect of the compounds on the energy expenditure of mice was demonstrated by means of the following procedure.

Female CFLP mice each weighing approximately 24 g, were given food and water *ad lib* before and during the experiment. The compounds were dissolved in water by addition of one mole of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content following the principles described by J B de V Weir, *J Physiol* (London) *109,* 1—9, (1949). The food intake of the mice was measured over this same period of 21 hours. The results are expressed as a percentage of the mean food intake or rate of energy expenditure of the mice dosed with water.

| Compound of Example No. | Dose (mg/kg po) | Mean Energy Expenditure (%) | Mean Food Intake (%) |
|---|---|---|---|
| Control | — | 100 | 100 |
| 1 | 26.1 | 129 | 98 |
| 1a | 2.61 | 120 | 94 |
| 2 | 26.9 | 127 | 98 |
| 3 | 25.6 | 125 | 83 |
| 4 | 24.6 | 110 | 76 |
| 5 | 30.2 | 134 | 102 |
| 6 | 28.9 | 97 | 98 |
| 7 | 28.3 | 125 | 109 |
| 8 | 31.6 | 135 | 97 |
| 9 | 28.3 | 129 | 109 |
| 10 | 27.3 | 118 | 89 |
| 11 | 25.3 | 148 | 79 |
| 12 | 24.2 | 120 | 99 |
| 13 | 29.5 | 139 | 78 |
| 14 | 23.9 | 141 | 111 |
| 15 | 26.9 | 117 | 77 |
| 16 | 23.4 | 141 | 91 |
| 17 | 22.0 | 114 | 87 |
| 18 | 26.3 | 131 | 80 |
| 19 | 27.3 | 135 | 94 |
| 20 | 25.3 | 115 | 86 |

| Compound of Example No. | Dose (mg/kg po) | Mean Energy Expenditure (%) | Mean Food Intake (%) |
|---|---|---|---|
| 21 | 23.6 | 119 | 102 |
| 22 | 24.3 | 124 | 84 |
| 23 | 6.5 | 109 | 112 |
| 24 | 6.6 | 139 | 102 |
| 25 | 25.5 | 117 | 87 |
| 26 | 26.4 | 125 | 63 |
| 27 | 24.2 | 143 | 100 |
| 28 | 21.8 | 135 | 108 |
| 29 | 21.8 | 123 | 107 |
| 30 | 22.6 | 125 | 95 |
| 31 | 24.3 | 100 | 59 |
| 33 | 26.3 | 131 | 80 |
| 34 | 18.4 | 117 | 103 |
| 35 | 23.1 | 95 | 83 |
| 36 | 22.7 | 132 | 78 |
| 38 | 19.2 | 132 | 81 |

(III) *Cardiac activity*

Rat hearts were perfused by the Langendorff procedure as follows:—

Hearts were dissected free within 30 seconds of death and reverse perfused via the aorta and coronary vessels with Krebs-Ringer bicarbonate solution (pH 7.4, 37°C) gassed with 95% oxygen:5% carbon dioxide at a flow rate between 8—12 $cm^3$/minute. Responses were observed after injection of drug dissolved in isotonic saline into the perfusion media. Heart rate and tension were displayed on an Ormed MX2P recorder via a tension transducer and heart ratemeter.

Results are expressed as a percentage of the maximum response due to salbutamol.

| Compound of Example No. | Dose added to perfusate ($\mu g$) | Heart Tension | Heart Rate |
|---|---|---|---|
| Salbutamol | — | 100 | 100 |
| 1 | 10 | 7 | 0 |
| 2 | 10 | 0 | 0 |
| 3 | 10 | 4 | 38 |
| 7 | 10 | 18 | 73 |
| 8 | 10 | 20 | 73 |
| 9 | 10 | 53 | 73 |
| 10 | 10 | 33 | 14 |
| 11 | 10 | 60 | 45 |
| 14 | 30 | 0 | 0 |
| 18 | 30 | 7 | 12 |
| 20 | 30 | 0 | 12 |
| 21 | 30 | 24 | 22 |
| 22 | 30 | 22 | 57 |

(IV) *Anti-obesity activity*

The compounds were administered by oral gavage in water or carboxymethyl-cellulose suspension to genetically obese mice daily for 28 days. At the end of the time the carcass composition was determined. The results obtained were as follows:—

| Compound of Example No. | Dose mg/kg p.o. | g Lipid/Mouse | |
|---|---|---|---|
| | | Treated | Control |
| 1a | 13.1 | 12.54 | 19.13 |
| 8 | 15.8 | 17.91 | 19.89 |
| 14 | 3.61 | 19.83 | 22.90 |

(V) *Platelet aggregation inhibition activity*

Collagen-induced platelet aggregation in human whole blood *in vitro*.

Collagen added to stirred citrated blood causes platelet aggregation. As the platelets aggregate, the concentration of single platelets falls. Hence the aggregation response can be quantified as a per cent fall in single platelet count. Inhibitors of aggregation, such as aspirin and dipyridamole, reduce the response to collagen.

(V) *Platelet aggregation inhibition activity (Cont)*

Blood drawn from volunteers who denied taking aspirin within the previous seven days was mixed with 0.1 vol 129 mM trisodium citrate, dispensed into 0.5 ml aliquots and kept at 25°. Collagen (Hormon-Chemie, Munich) or 154 mM NaCl (control) was added to each aliquot of blood stirred at 1100 rpm in an aggregometer at 37° after 3 mins pre-incubation with compound or appropriate solvent. Aggregates were fixed by addition of 0.5 vol 4.5% formaldehyde or 0.05 vol phosphate-buffered glutaraldehyde. Single platelets were counted electronically. Responses, expressed as per cent fall in platelet count, were obtained to a range of collagen concentrations. The concentration producing 50% maximal effect (EC50) was estimated from $\log_{10}$ concentration-response curves. Dose-ratios were calculated by dividing the EC50 obtained in the presence of compound under test by the control EC50.

Results are summarised in the table.

| Compound | Final Concentration in Whole Blood (μM) | Dose-Ratio |
|---|---|---|
| Aspirin | 200 | 2.4 = 0.3 (3) |
| Dipyridamole | 200 | 3.7 = 1.1 (4) |
| Example 24 | 100 | 5.9 |
| Example 8 | 100 | 4.8 |

(VI) *Anti-inflammatory activity*

The method used is based on that described by G. Tonelli *et al* (Endocrinology, *77,* 625—634, 1965). An inflammation is induced in the rat ear by the application of 50 μl of a 1% solution of croton oil in tetrahydrofuran, test compounds being dissolved in the irritant vehicle. After 6 hours the inflammation is assessed by killing the animals and weighing the ears. Topical anti-inflammatory activity of a compound is generally considered to be shown when a significant (5% level) reduction in ear weight is seen compared to non-drug treated control.

| Compound of Example No. | Dose mg/rat ear | Activity |
|---|---|---|
| 24 | 1.35 | Active |

*Toxicity*

No toxicity was observed during the above experiments.

**Claims**

1. A compound of formula (I):

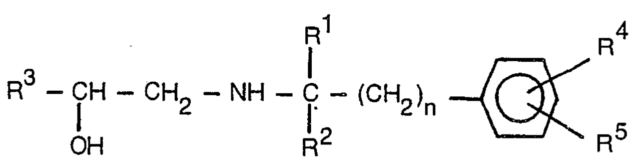

(I)

and salts and *in vivo* hydrolysable acyl derivatives thereof, wherein

$R^1$ is hydrogen or methyl;

$R^2$ is hydrogen or methyl;

$R^3$ is phenyl unsubstituted or substituted with one or two of the following:— fluorine, chlorine, bromine, trifluoromethyl, $C_{1-6}$ straight or branched chain alkyl or $C_{1-6}$ straight or branched chain alkoxy; or is benzofuran-2-yl;

$R^5$ is a hydrogen, halogen, hydroxy, lower alkyl or lower alkoxy;

$R^4$ is moiety

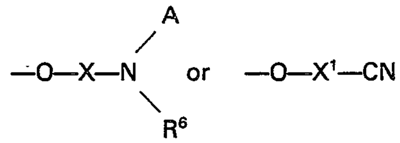

wherein X is $C_{2-10}$ alkylene which may be straight or branched provided that the oxygen and nitrogen are separated by at least two carbon atoms,

$X^1$ is $C_{1-9}$ alkylene which may be straight or branched,

A is hydrogen; $C_{1-6}$ straight or branched alkyl or benzyl optionally substituted with halogen, lower alkyl or lower alkoxy.

$R^6$ is hydrogen; $C_{1-6}$ straight or branched alkyl; $C_{1-6}$ straight or branched alkylsulphonyl or

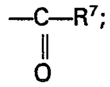

$R^7$ is $C_{1-6}$ straight or branched alkyl; $C_{1-6}$ straight or branched alkoxy; amino, mono- or di- ($C_{1-6}$ straight or branched alkyl amino; or

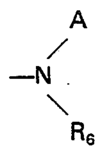

is a 5, 6 or 7 membered cyclic amino group optionally containing a second heteroatom selected from oxygen, nitrogen and sulphur

and n is 1 or 2.

2. A compound as claimed in claim 1 wherein the carbon atom bearing the hydroxyl group and $R^3$ has the *R*-absolute configuration.

3. A compound as claimed in claim 1 and selected from

N-[2-(4-(2-Methylaminoethoxy)phenyl)-1-methylethyl]-2-hydroxy-2-(3-trifluoromethylphenyl)ethanamine;

N-[2-(4-(2-Dimethylaminoethoxy)phenyl)-1-methylethyl]-2-hydroxy-2-(3-trifluoromethylphenyl)ethanamine;

N-[2-(4-(2-Methylsulphonylaminoethoxy)phenyl-1-methylethyl]-2-hydroxy-2-(3-trifluoromethylphenyl)ethanamine;

N-[2-(4-(2-Methylaminoethoxy)phenyl)-1-methylethyl]-2-hydroxy-2-(2-benzofuranyl)ethanamine;

N-[2-(3-Fluoro-4-(2-methylaminoethoxy)phenyl)-1-methylethyl]-2-hydroxy-2-(3-chlorophenyl)ethanamine;

N-[2-(4-(2-Methylaminoethoxy)phenyl)-1-methylethyl]-2-hydroxy-2-(3-ethylphenyl)ethanamine;

N-[2-(4-(2-Methylaminoethoxy)phenyl)-1-methylethyl]-2-hydroxy-2-(2-fluorophenyl)ethanamine;

N-[2-(4-(2-Methylaminoethoxy)phenyl)-1-methylethyl]-2-hydroxy-2-(3-bromophenyl)ethanamine;

N-[2-(4-(2-Methylaminoethoxy)phenyl-1-methylethyl]-2-hydroxy-2-(3-fluorophenyl)ethanamine;

N-[2-(4-(2-Methylaminoethoxy)phenyl-1-methylethyl]-2-hydroxy-2-phenylethanamine;

N-[2-(4-(2-Methylaminoethoxy)phenyl-1-methylethyl]-2-hydroxy-2-(2-fluoro-3-chlorophenyl)ethanamine;

N-[2-(4-(2-Methylaminoethoxy)phenyl)-1-methylethyl]-2-hydroxy-2-(4-chlorophenyl)ethanamine;

N-[2-(4-(2-Benzylaminoethoxy)phenyl)-1-methylethyl]-2-hydroxy-2-(3-chlorophenyl)ethanamine;

N-[2-(4-(2-(1-Piperidine)ethoxy)phenyl)-1-methylethyl]-2-hydroxy-2-(3-chlorophenyl)ethanamine;

N-[2-(4-(2-(1-Homopiperidine)ethoxy)phenyl)1)methylethyl]-2-hydroxy-2-(3-trifluoromethylphenyl)ethanamine;

N-[2-(4-(2-Ethoxycarbonylaminoethoxy)phenyl)-1-methylethyl]-2-hydroxy-2-(3-chlorophenyl)ethanamine;

N-[2-(4-(2-Methylaminocarbonylaminoethoxy)phenyl)-1-methylethyl]-2-hydroxy-2-(3-trifluoromethylphenyl)ethanamine;

N-[2-(4-(2-Benzylaminoethoxy)phenyl)-1-methylethyl)-2-hydroxy-2-(3-trifluoromethylphenyl)ethanamine;

N-[2-(4-(2-Aminoethoxy)phenyl)-1-methylethyl]-2-hydroxy-2-(3-trifluoromethylphenyl)ethanamine;
N-[2-(4-(2-Ethoxycarbonylaminoethoxy)phenyl)-1-methylethyl]-2-hydroxy-2-(3-trifluoromethylphenyl)ethanamine;
N-[2-(4-(2-Acetylaminoethoxy)phenyl)-1-methylethyl]-2-hydroxy-2-(3-trifluoromethylphenyl)ethanamine;
N-[2-(4-(2-Methylaminoethoxy)phenyl)-1-methylethyl]-2-hydroxy-2-(3-chlorophenyl)ethanamine;
N-[2-(4-(2-Methylaminoethoxy)phenyl-1-methylethyl]-2-hydroxy-2-(3-trifluoromethylphenyl)ethanamine, (RS,SR) diastereoisomer;
N-[2-(4-(2-Methylaminoethoxy)phenyl)-1-methylethyl]-2-hydroxy-2-(3-trifluoromethylphenyl)ethanamine (RR,SS) diastereoisomer;
N-[2-(4-(2-(1-Pyrrolidine)ethoxy)phenyl)-1-methylethyl]-2-hydroxy-2-(3-trifluoromethylphenyl)ethanamine;
N-[2-(4-(2-(1-Piperidine)ethoxy)phenyl)-1-methylethyl]-2-hydroxy-2-(3-trifluoromethylphenyl)ethanamine;
N-[2-(4-(2-Ethylaminoethoxy)phenyl)-1-methylethyl]-2-hydroxy-2-(3-trifluoromethylphenyl)ethanamine;
N-[2-(4-(2-Methylaminoethoxy)phenyl)-1-methylethyl]-2-hydroxy-2-(3-chlorophenyl)ethanamine, (RR,SS) diastereoisomer;
N-[2-(3-(2-Methylaminoethoxy)phenyl)-1-methylethyl]-2-hydroxy-2-(3-chlorophenyl)ethanamine;
N-[2-(4-(2-(1-Morpholino)ethoxy)phenyl)-1-methylethyl]-2-hydroxy-2-(3-trifluoromethylphenyl)ethanamine;
N-[2-(4-(2-Benzylaminopropoxy)phenyl)-1-methylethyl]-2-hydroxy-2-(3-trifluoromethylphenyl)ethanamine;
N-[2-(4-(2-Aminopropoxy)phenyl)-1-methylethyl]-2-hydroxy-2-(3-trifluoromethylphenyl)ethanamine;
N-[2-(4-(6-Methylaminohexyloxy)phenyl)-1-methylethyl]-2-hydroxy-2-(3-trifluoromethylphenyl)ethanamine;
N-[2-(4-(2-Aminoethoxy)phenyl)ethyl]-2-hydroxy-2-(3-trifluoromethylphenyl)ethanamine;
N-[3-(4-(2-Dimethylaminoethoxy)phenyl)-1,1-dimethylpropyl]-2-hydroxy-2-phenylethanamine;
N-[2-(4-(2-Dimethylaminoethoxy)phenyl)-1-methylethyl]-2-hydroxy-2-(3-trifluoromethylphenyl)ethanamine;
N-[2-(4-Cyanomethoxy)phenyl)ethyl]-2-hydroxy-2-(3-trifluoromethylphenyl)ethanamine;
N-[2-(4-Cyanomethoxy)phenyl)-1-methylethyl]-2-hydroxy-2-(3-chlorophenyl)ethanamine;
and salts thereof,

4. A process for producing a compound of formula (I) as defined in claim 1 or a salt thereof, which process comprises (a) reducing an oxo-group and/or a double bond and/or a moiety reducible to a group $R^4$ of a compound of formula (II):

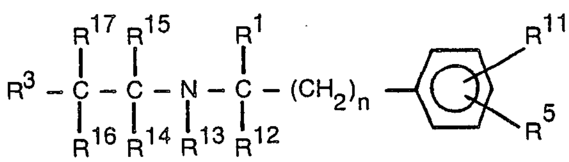
(II)

wherein $R^1$, $R^3$, $R^5$ and n are as defined in relation to formula (I)
$R^{11}$ is $R^4$ or a moiety reducible to a group $R^4$ as defined in relation to formula (I);
$R^{12}$ is a group $R^2$ as defined in relation to formula (I) or together with $R^{13}$ forms a bond;
$R^{13}$ is hydrogen or benzyl; or together with $R^{12}$ or $R^{14}$ forms a bond;
$R^{14}$ is hydrogen or together with $R^{15}$ forms an oxo-group or together with $R^{13}$ forms a bond;
$R^{15}$ is hydrogen or together with $R^{14}$ forms an oxo-group;
$R^{16}$ is hydroxyl or together with $R^{17}$ forms an oxo-group;
$R^{17}$ is hydrogen or together with $R^{16}$ forms an oxo-group,
provided that there is no more than one oxo-group and no more than one bond represented by any of $R^{12}$ to $R^{17}$, and optionally thereafter forming a salt of the compound of formula (I) also formed and/or converting the compound of formula (I) so formed into a further compound of formula )I),
or (b) reacting a compound of formula (III):

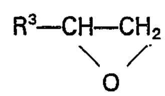
(III)

or a compound of formula (VIIID)

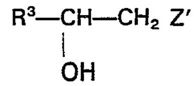

(VIIID)

wherein Z′ is a leaving group such as halogen or preferably tosylate with a compound of formula (IV):

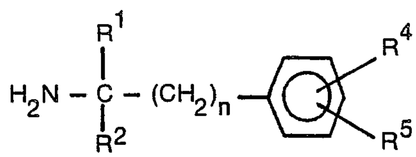

(IV)

wherein $R^1$, $R^2$, $R^3$, $R^4$, $R^5$ and n are as defined in relation to formula (I), and optionally thereafter forming a salt of the compond of formula (I) so formed or (c) reacting an alkali metal salt of a compound of formula (V):

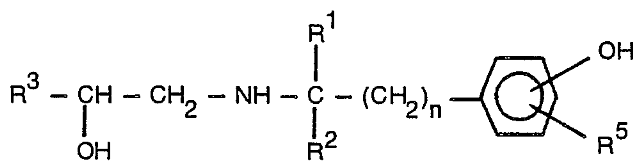

(V)

with a compound of formula (VI):

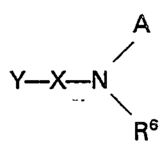

(VI)

wherein $R^1$, $R^2$, $R^3$, $R^5$, $R^6$, A, X and n are as defined in relation to formula (I), and Y is a leaving group.

5. A process as claimed in claim 4(a) wherein the reduction is effected using hydrogen and palladium catalyst in a suitable solvent.

6. A process as claimed in claim 4(a) wherein the reduction is effected using sodium borohydride in a suitable solvent.

7. A pharmaceutical formulation comprising a compound of formula (I) as defined in claim 1 and a pharmaceutically acceptable carrier therefor.

8. A pharmaceutical formulation as claimed in claim 8 in unit dosage form.

9. A process for producing a formulation as claimed in claim 7 which comprises bringing into association the compound of formula (I) and the carrier.

10. A compound as claimed in claim 1 for use in treating the human or animal body.

**Patentansprüche**

1. Eine Verbindung der Formel (I):

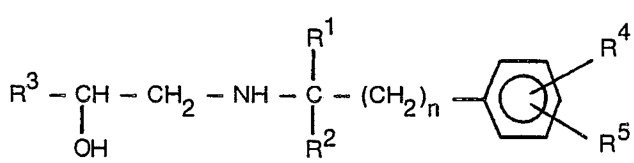

(I)

und Salze und *in vivo* hydrolysierbare Acylderivate derselben, in welcher

$R^1$ Wasserstoff oder Methyl ist;

$R^2$ Wasserstoff oder Methyl ist;

$R^3$ Phenyl, unsubstituiert oder substituiert durch eine oder zwei der folgenden Elemente bzw. Gruppen:Fluor, Chlor, Brom, Trifluormethyl, geradkettiges oder verzweigtkettiges $C_{1-6}$-Alkyl oder geradkettiges oder verzweigtkettiges $C_{1-6}$-Alkoxy; oder Benzofuran-2-yl darstellt;

$R^5$ Wasserstoff, Halogen, Hydroxy, niedriges Alkyl oder niedriges Alkoxy bedeutet;

$R^4$ die Gruppe

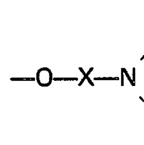 oder $-O-X^1-CN$

darstellt, in welcher X $C_{2-10}$-Alkylen ist, welches geradkettig oder verzweigtkettig sein kann, vorausgesetzt daß Sauerstoff und Stickstoff durch mindestens 2 Kohlenstoffatomen voneinander getrennt sind,

$X^1$ $C_{1-9}$-Alkylen ist, welches geradkettig oder verzweigtkettig sein kann;

A Wasserstoff, gerad- oder verzweigkettiges $C_{1-6}$-Alkyl oder Benzyl, gegebenenfalls substituiert durch Halogen, niedriges Alkyl oder niedriges Alkoxy, bedeutet;

$R_6$ Wasserstoff, gerad- oder verzweigtkettiges $C_{1-6}$-Alkyl; gerad- oder verzweigtkettiges $C_{1-6}$Alkylsulphonyl oder eine Gruppe

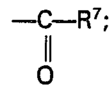

darstellt;

$R^7$ ein gerad- oder verzweigtkettiges $C_{1-6}$-Alkyl; gerad- oder verzweigtkettiges $C_{1-6}$-Alkoxy; Amino, Mono- oder Di($C_{1-6}$ — gerad- oder verzweigkettiges Alkyl)amino bedeutet;

oder 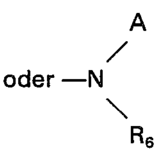

eine 5-, 6- oder 7-gliedrige cyclische Aminogruppe ist, welche gegebenenfalls ein zweites Heteroatom, ausgewählt aus Sauerstoff, Stickstoff und Schwefel, enthält, und

n den Wert 1 oder 2 hat.

3. Eine Verbindung wie in Anspruch 1 beansprucht, ausgewählt aus

N-[2-(4-(2-Methylaminoäthoxy)phenyl-1-methyläthyl]-2-hydroxy-2-(3-trifluormethylphenyl)äthanamin;

N-[2-(4-(2-Dimethylaminoäthoxy)phenyl)-1-methyläthyl]-2-hydroxy-2-(3-trifluormethylphenyl)äthanamin;

N-[2-(4-(2-Methylsulphonylaminoäthoxy)phenyl-1-methyläthyl]-2-hydroxy-2-(3-trifluormethylphenyl)äthanamin;

N-[2-(4-(2-Methylaminoäthoxy)phenyl)-1-methyläthyl]-2-hydroxy-2-(2-benzofuranyl)äthanamin;

N-[2-(3-Fluor-4-(2-methylaminoäthoxy)phenyl-1-methyläthyl]-2-hydroxy-2-(3-chlorphenyl)äthanamin;

N-[2-(4-(2-Methylaminoäthoxy)phenyl)-1-methyläthyl]-2-hydroxy-2-(3-äthylhenyl)äthanamin;

N-[2-(4-(2-Methylaminoäthoxy)phenyl)-1-methyläthyl]-2-hydroxy-2-(2-fluorphenyl)äthanamin;

N-[2-(4-(2-Methylaminoäthoxy)phenyl)-1-methyläthyl-2-hydroxy-2-(3-bromphenyl)äthanamin;

N-[2-(4-(2-Methylaminoäthoxy)phenyl-1-methyläthyl]-2-hydroxy-2-(3-fluorphenyl)äthanamin;

N-[2-(4-(2-Methylaminoäthoxy)phenyl-1-methyläthyl]-2-hydroxy-2-phenyläthanamin;

N-[2-(4-(2-Methylaminoäthoxy)phenyl)-1-methyläthyl]-2-hydroxy-2-(2-fluor-3-chlorphenyl)äthanamin;

N-[2-(4-(2-Methylaminoäthoxy)phenyl)-1-methyläthyl]-2-hydroxy-2-(4-chlorphenyl)äthanamin;

N-[2-(4-(2-Benzylaminoäthoxy)phenyl)-1-methyläthyl]-2-hydroxy-2-(3-chlorphenyl)äthanamin;

N-[2-(4-(1-Piperidin)äthoxy)phenyl-1-methyläthyl]-2-hydroxy-2-(3-chlorphenyl)äthanamin;

N-[2-(4-(2-(1-Homopiperidin)äthoxy)phenyl)-1-methyläthyl]-2-hydroxy-2-(3-trifluormethylphenyl)äthanamin;

N-[2-(4-(2-Äthoxycarbonylaminoäthoxy)phenyl)-1-methyläthyl]-2-hydroxy-2-(3-chlorphenyl)äthanamin);

N-[2-(4-(2-Methylaminocarbonylaminoäthoxy)phenyl)-1-methyläthyl]-2-hydroxy-2-(3-trifluormethylphenyl)äthanamin;

N-[2-(4-(2-Benzylaminoäthoxy)phenyl)-1-methyläthyl]-2-hydroxy-2-(3-trifluormethylphenyl)äthanamin;

N-[2-(4-(2-Aminoäthoxy)phenyl)-1-methyläthyl]-2-hydroxy-2-(3-trifluormethylphenyl)äthanamin;

N-[2-(4-(2-äthoxycarbonylaminoäthoxy)phenyl)-1-methyläthyl]-2-hydroxy-2-(3-trifluormethylphenyl)äthanamin;

N-[2-(4-(2-Acetylaminoäthoxy)phenyl)-1-methyläthyl]-2-hydroxy-2-(3-trifluormethylphenyl)-äthanamin;

N-[2-(4-(2-Methylaminoäthoxy)phenyl)-1-methyläthyl]-2-hydroxy-2-(3-chlorphenyl)äthanamin;

N-[2-(4-(2-Methylaminoäthoxy)phenyl)-1-methyläthyl]-2-hydroxy-2-(3-trifluormethylphenyl)-äthanamin, (RS,SR) Diastereoisomer;

N-[2-(4-(2-Methylaminoäthoxy)phenyl)-1-methyläthyl]-2-hydroxy-2-(3-trifluormethylphenyl)-äthanamin (RR,SS) Diastereoisomer;

N-[2-(4-(2-(1-Pyrrolidin)äthoxy)phenyl)-1-methyläthyl]-2-hydroxy-2-(3-trifluormethylphenyl)-äthanamin;

N-[2-(4-(2-(1-Piperidin)äthoxy)phenyl)-1-methyläthyl]-2-hydroxy-2-(3-trifluormethylphenyl)-äthanamin;

N-[2-(4-(2-äthylaminoäthoxy)phenyl)-1-methyläthyl]-2-hydroxy-2-(3-trifluormethylphenyl)-äthanamin;

N-[2-(4-(2-Methylaminoäthoxy)phenyl)-1-methyläthyl]-2-hydroxy-2-(3-chlorphenyl)äthanamin (RR,SS) Diastereoisomer;

N-[2-(3-(2-Methylaminoäthoxy)phenyl)-1-methyläthyl]-2-hydroxy-2-(3-chlorphenyl)äthanamin;

N-[2-(4-(2-(1-Morpholino)äthoxy)phenyl)-1-methyläthyl]-2-hydroxy-2-(3-trifluormethylphenyl)-äthanamin;

N-[2-(4-(2-Benzylaminopropoxy)phenyl)-1-methyläthyl]-2-hydroxy-2-(3-trifluormethylphenyl)-äthanamin;

N-[2-(4-(2-Aminopropoxy)phenyl)-1-methyläthyl]-2-hydroxy-2-(3-trifluormethylphenyl)äthanamin;

N-[2-(4-(6-Methylaminohexyloxy)phenyl)-1-methyläthyl]-2-hydroxy-2-(3-trifluormethylphenyl)-äthanamin;

N-[2-(4-(2-Aminoäthoxy)phenyl)-äthyl]-2-hydroxy-2-(3-trifluormethylphenyl)äthanamin;

N-[3-(4-(2-Dimethylaminoäthoxy)phenyl)-1,1-dimethylpropyl]-2-hydroxy-2-phenyläthanamin;

N-[2-(4-(2-Dimethylaminoäthoxy)phenyl)-1-methyläthyl]-2-hydroxy-2-(3-trifluormethylphenyl)-äthanamin;

N-[2-(4-Cyanomethoxy)phenyl)äthyl]-2-hydroxy-2-(3-trifluormethylphenyl)äthanamin;

N-[2-(4-Cyanomethoxy)phenyl)-1-methyläthyl]-2-hydroxy-2-(3-chlorphenyl)äthanamin;

und Salze davon.

4. Ein Verfahren zur Herstellung einer Verbindung der Formel (I) wie in Anspruch 1 definiert oder ein Salz derselbe, welches Verfahren umfaßt (a) die Reduktion einer Oxo-Gruppe und/oder einer Doppelbindung und/oder eines auf eine Gruppe $R^4$ reduzierbaren Molekülteils einer Verbindung der Formel (II):

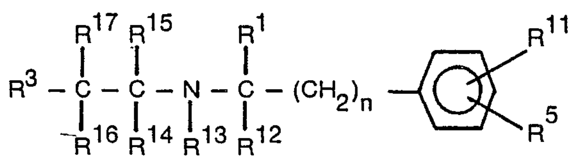

(II)

in welcher $R^1$, $R^3$, $R^5$ und n wie in bezug auf Formel (I) definiert sind,

$R^{11}$ $R^4$ oder einen auf Gruppe $R^4$, wie in bezug auf Formel (I) definiert, reduzierbaren Molekülteil darstellt;

$R^{12}$ eine Gruppe $R^2$, wie in bezug auf Formel (I) definiert, ist oder zusammen mit $R^{13}$ eine Bindung bildet;

$R^{13}$ Wasserstoff oder Benzyl ist; oder zusammen mit $R^{12}$ oder $R^{14}$ eine Bindung bildet;

$R^{14}$ Wasserstoff ist oder zusammen mit $R^{15}$ eine Oxo-Gruppe oder zusammen mit $R^{13}$ eine Bindung bildet;

$R^{15}$ Wasserstoff ist oder zusammen mit $R^{14}$ eine Oxo-Gruppe bildet;

$R^{16}$ Hydroxyl ist oder zusammen mit $R^{17}$ eine Oxo-Gruppe bildet;

$R^{17}$ Wasserstoff ist oder zusammen mit $R^{16}$ eine Oxo-Gruppe bildet;

vorausgesetzt, daß nicht mehr als eine Oxo-Gruppe und nicht mehr als eine Bindung durch eine der Gruppen $R^{12}$ bis $R^{17}$ dargestellt sind, und gewünschtenfalls anschließend die Bildung eines Salzes der so gebildeten Verbindung der Formel (I) und/oder Umwandlung der so gebildeten Verbindung der Formel (I) in eine weitere Verbindung der Formel (I),

oder (b) die Umsetzung einer Verbindung der Formel (III):

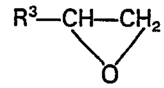

(III)

oder einer Verbindung der Formel (VIIID):

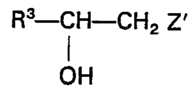

(VIIID)

in welcher Z' eine abspaltbare Gruppe ist, wie z.B. Halogen oder vorzugsweise Tosylat, mit einer Verbindung der Formel (IV):

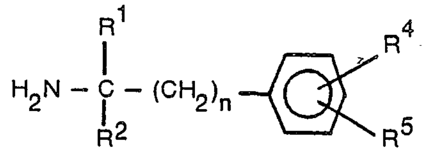

(IV)

in welcher $R^1$, $R^2$, $R^3$, $R^4$, $R^5$ und n wie in bezug auf Formel (I) definiert sind, und gewünschtenfalls anschließend die Bildung eines Salzes der der so gebildeten Verbindung der Formel (I) oder (c) die Umsetzung eines Alkalimetalls einer Verbindung der Formel (V):

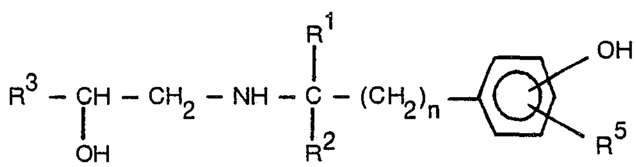

(V)

mit einer Verbindung der Formel (VI):

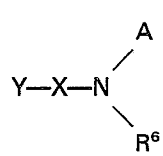

(VI)

in welcher $R^1$, $R^2$, $R^3$, $R^5$, $R^6$, A, X und n wie in bezug auf Formel (I) definiert sind, und Y eine abspaltbare Gruppe ist.

5. Ein Verfahren wie in Anspruch 4(a) beansprucht, in welchem die Reduktion unter Verwendung von Wasserstoff und Palladiumkatalysatoren in einem geeigneten Lösungsmittel durchgeführt wird.

6. Ein Verfahren wie in Anspruch 4(a) beansprucht, in welchem die Reduktion unter Verwendung von Natriumborhydrid in einem geeigneten LÖsungsmittel durchgeführt wird.

7. Eine pharmazeutische Formulierung umfassend eine Verbindung der Formel (I) wie in Anspruch 1 definiert und einen pharmazeutisch verträglichen Träger hierfür.

8. Eine pharmazeutische Formulierung wie in Anspruch 7 beansprucht in Einzeldosisform.

9. Ein Verfahren zur Herstellung einer Formulierung wie in Anspruch 7 beansprucht, welches das in-Verbindung-Bringen der Verbindung der Formel (I) mit dem Träger umfaßt.

10. Eine Verbindung wie in Anspruch 1 beansprucht, zur Verwendung bei der Behandlung des menschlichen oder tierischen Körpers.

**Revendications**

1. Composé de formule (I)

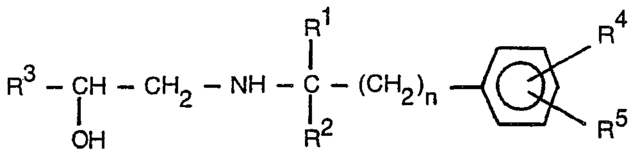

(I)

et ses sels et dérivés acyles hydrolysables in vivo, dans laquelle

$R^1$ est un atome d'hydrogéne ou un groupe méthyle;

$R^2$ est un atome d'hydrogéne ou un groupe méthyle;

$R^3$ est un groupe phényle non-substitué ou substitué avec un ou deux des atomes ou groupes suivants: fluor, chlore, brome, trifluorométhyle, alcoyle en $C_{1-6}$ à chaîne droite ou ramifiée, ou alkoxy en $C_{1-6}$ à chaîne droite ou ramifiée il est un groupe benzo-furan-2-yle;

$R^5$ est un atome d'hydrogène, d'halogène, un groupe hydroxy, alcoyle inférieur ou alcoxy inférieur;

$R^4$ est une partie

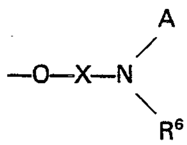 ou $—O—X^1—CN$

dans laquelle

X est un groupe alcoylène en $C_{2-10}$ qui peut être droit ou ramifié, à condition que les atomes d'oxygène et d'azote soient séparés par deux atomes de carbone au moins;

$X^1$ est un groupe alcoylène en $C_{1-9}$ à chaîne droite ou ramifiée;

A est un atome d'hydrogène, un groupe alcoyle en $C_{1-6}$ à chaîne droite ou ramifiée ou benzyle éventuellement substitué avec un atome d'halogène, un groupe alcoyle inférieur ou alcoxy inférieur;

$R^6$ est un atome d'hydrogène, un groupe alcoyle en $C_{1-6}$ à chaîne droite ou ramifiée, alcoylsulfonyle en $C_{1-6}$ à chaîne droite ou ramifiée ou

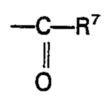

$R^7$ est un groupe alcoyle en $C_{1-6}$ à chaîne droite ou ramifiée, alcoxy en $C_{1-6}$ à chaîne droite ou ramifiée, amino, mono- ou di-(alcoyle en $C_{1-6}$ à chaîne droite ou ramifiée) amino;

ou 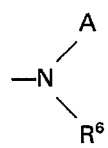

est un groupe aminocyclique à 5, 6 ou 7 chaînons contenant éventuellement un second hétéroatome choisi parmi l'oxygène, l'azote et le soufre; et n est 1 ou 2.

2. Composé suivant la revendication 1, caractérisé en ce que l'atome de carbone portant le groupe hydroxy et $R^3$ a la configuration *R* absolue.

3. Composé suivant la revendication 1 et choisi parmi
la N-[2-(4-(2-méthylaminoéthoxy)phényl)-1-méthyléthyl]-2-hydroxy-2-(3-trifluorométhylphényl)-éthanamine,
la N-[2-(4-(2-diméthylaminoéthoxy)phényl)-1-méthyléthyl]-2-hydroxy-2-(3-trifluorométhylphényl)-éthanamine,
la N-[2-(4-(2-méthylsulfonylaminoéthoxy)phényl)-1-méthyléthyl]-2-hydroxy-2-(3-trifluorométhyl-phényl)éthanamine,
la N-[2-(4-(2-méthylaminoéthoxy)phényl)-1-méthyléthyl]-2-hydroxy-2-(2-benzofuranyl)éthanamine,
la N-[2-(3-fluoro-4-(2-méthylaminoéthoxy)phényl)-1-méthyléthyl]-2-hydroxy-2-(3-chlorophényl)-éthanamine,
la N-[2-(4-(2-méthylaminoéthoxy)phényl)-1-méthyléthyl]-2-hydroxy-2-(3-éthylphényl)éthanamine,
la N-[2-(4-(2-méthylaminoéthoxy)phényl)-1-méthyléthyl]-2-hydroxy-2-(2-fluorophényl)éthanamine,
la N-[2-(4-(2-méthylaminoéthoxy)phényl)-1-méthyléthyl]-2-hydroxy-2-(3-bromophényl)éthanamine,
la N-[2-(4-(2-méthylaminoéthoxy)phényl)-1-méthyléthyl]-2-hydroxy-2-(3-fluorophényl)éthanamine,
la N-[2-(4-(2-méthylaminoéthoxy)phényl)-1-méthyléthyl]-2-hydroxy-2-phényléthanamine,
la N-[2-(4-(2-méthylaminoéthoxy)phényl)-1-méthyléthyl]-2-hydroxy-2-(2-fluoro-3-chlorophényl)-éthanamine,
la N-[2-(4-(2-méthylaminoéthoxy)phényl)-1-méthyléthyl]-2-hydroxy-2-(4-chlorophényl)éthanamine,
la N-[2-(4-(2-benzylaminoéthoxy)phényl)-1-méthyléthyl]-2-hydroxy-2-(3-chlorophényl)éthanamine,
la N-[2-(4-(2-1-pipéridine)éthoxy)phényl)-1-méthyléthyl]-2-hydroxy-2-(3-chlorophényl)éthanamine,
la N-[2-(4-(2-(1-homopipéridine)éthoxy)phényl)-1-méthyléthyl]-2-hydroxy-2-(3-trifluorométhyl-phényl)éthanamine,
la N-[2-(4-(2-éthoxycarbonylaminoéthoxy)phényl)-1-méthyléthyl]-2-hydroxy-2-(3-chlorophényl)-éthanamine,
la N-[2-(4-(2-méthylaminocarbonylaminoéthoxy)phényl)-1-méthyléthyl]-2-hydroxy-2-(3-trifluorométhylphényl)éthanamine,
la N-[2-(4-(2-benzylaminoéthoxy)phényl)-1-méthyléthyl]-2-hydroxy-2-(3-trifluorométhylphényl)-éthanamine,
la N-[2-(4-(2-aminoéthoxy)phényl)-1-méthyléthyl]-2-hydroxy-2-(3-trifluorométhylphényl)-éthanamine,
la N-[2-(4-(2-éthoxycarbonylaminoéthoxy)phényl)-1-méthyléthyl]-2-hydroxy-2-(3-trifluorométhyl-phényl)éthanamine,
la N-[2-(4-(2-acétylaminoéthoxy)phényl)-1-méthyléthyl]-2-hydroxy-2-(3-trifluorométhylphényl)-éthanamine,
la N-[2-(4-(2-méthylaminoéthoxy)phényl)-1-méthyléthyl]-2-hydroxy-2-(3-chlorophényl)éthanamine,
la N-[2-(4-(2-méthylaminoéthoxy)phényl)-1-méthyléthyl]-2-hydroxy-2-(3-trifluorométhylphényl)-éthanamine, diastéréoisomère (RS, SR).
la N-[2-(4-(2-méthylaminoéthoxy)phényl)-1-méthyléthyl]-2-hydroxy-2-(3-trifluorométhylphényl)-éthanamine, diastéréoisomère (RR,SS),
la N-[2-(4-(2-(1-pyrrolidine)éthoxy)phényl)-1-méthyléthyl]-2-hydroxy-2-(3-trifluorométhylphényl)-éthanamine,
la N-[2-(4-(2-(1-pipéridine)éthoxy)phényl)-1-méthyléthyl]-2-hydroxy-2-(3-trifluorométhylphényl)-éthanamine,
la N-[2-(4-(2-éthylaminoéthoxy)phényl)-1-méthyléthyl]-2-hydroxy-2-(3-trifluorométhylphényl)-éthanamine,
la N-[2-(4-(2-méthylaminoéthoxy)phényl)-1-méthyléthyl]-2-hydroxy-2-(3-chlorophényl)éthanamine, diastéréoisomère (RR,SS),
la N-[2-(3-(2-méthylaminoéthoxy)phényl)-1-méthyléthyl]-2-hydroxy-2-(3-chlorophényl)éthanamine,
la N-[2-(4-(2-(1-morpholino)éthoxy)phényl)-1-méthyléthyl]-2-hydroxy-2-(3-trifluorométhylphényl)-éthanamine,
la N-[2-(4-(2-benzylaminopropoxy)phényl)-1-méthyléthyl]-2-hydroxy-2-(3-trifluorométhylphényl)-éthanamine,
la N-[2-(4-(2-aminopropoxy)phényl)-1-méthyléthyl]-2-hydroxy-2-(3-trifluorométhylphényl)-éthanamine,
la N-[2-(4-(6-méthylaminohexyloxy)phényl)-1-méthyléthyl]-2-hydroxy-2-(3-trifluorométhylphényl)-éthanamine,
la N-[2-(4-(2-aminoéthoxy)phényl)éthyl]-2-hydroxy-2-(3-trifluorométhylphényl)éthanamine,
la N-[3-(4-(2-diméthylaminoéthoxy)phényl)-1,1-diméthylpropyl]-2-hydroxy-2-phényléthanamine,
la N-[2-(4-(2-diméthylaminoéthoxy)phényl)-1-méthyléthyl]-2-hydroxy-2-(3-trifluorométhylphényl)-éthanamine,
la N-[2-(4-cyanométhoxy)phényl)éthyl]-2-hydroxy-2-(3-trifluorométhylphényl)éthanamine,
la N-[2-(4-cyanométhoxy)phényl)-1-méthyléthyl]-2-hydroxy-2-(3-chlorophényl)éthanamine,
et leurs sels.

4. Procédé de production d'un composé de formule (I) tel que défini dans la revendication 1 ou d'un sel de celui-ci, caractérisé en ce qu'il comprend:

(a) la réduction d'un groupe oxo et/ou d'une double liaison et/ou d'une partie réductible en un groupe $R^4$ d'un composé de formule (II) ci-après:

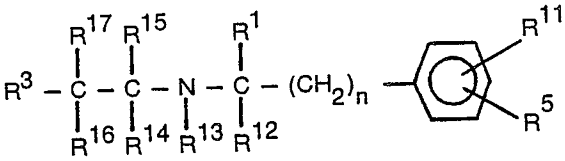

(II)

dans laquelle $R^1$, $R^3$, $R^5$ et n sont tels que défini à propos de la formule (I),

$R^{11}$ est un groupe $R^4$ ou une partie réductible en un groupe $R^4$ tel que défini à propos de la formule (I),

$R^{12}$ est un groupe $R^2$ tel que défini à propos de la formule (I) ou il forme une liaison avec $R^{13}$,

$R^{13}$ est un atome d'hydrogène ou un groupe benzyle, ou il forme une liaison avec $R^{12}$ ou $R^{14}$,

$R^{14}$ est un atome d'hydrogène ou il forme un groupe oxo avec $R^{15}$ ou une liaison avec $R^{13}$,

$R^{15}$ est un atome d'hydrogène ou il forme un groupe oxo avec $R^{14}$,

$R^{16}$ est un groupe hydroxy ou il forme un groupe oxo avec $R^{17}$,

$R^{17}$ est un atome d'hydrogène ou il forme un groupe oxo avec $R^{16}$ à condition qu'il n'y ait pas plus d'un groupe oxo et pas plus d'une liaison représentés par de quelconques groupes $R^{12}$ à $R^{17}$ et éventuellement, la formation ultérieure d'un sel du composé de formule (I) ainsi formé et/cu la conversion du compose de formule (I) ainsi formé en un autre composé de formule (I), ou

(b) la réaction d'un composé de formule (III)

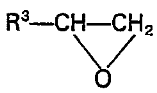

(III)

ou d'un composé de formule (VIIID)

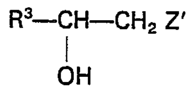

(VIIID)

où Z' est un groupe mobile, comme un atome d'halogène ou de préférence le groupe tosylate, avec un composé de formule (IV)

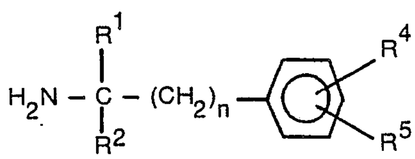

(IV)

dans laquelle $R^1$, $R^2$, $R^3$, $R^4$, $R^5$ et n sont tels que définis à propos de la formule (I) et éventuellement ensuite la formation d'un sel du composé de formule (I) ainsi formé, ou

(c) la réaction d'un sel de métal alcalin d'un composé de de formule (V)

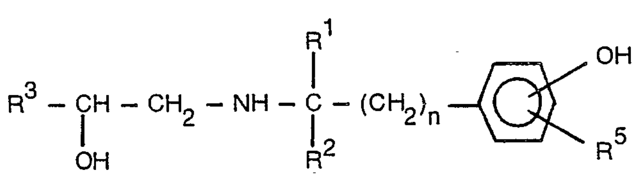

(V)

avec un composé de formule (VI)

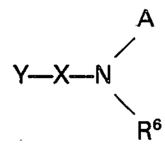

(VI)

où $R^1$, $R^2$, $R^3$, $R^5$, $R^6$, A, X et n sont tels que définis à propos de la formule (I), et Y est un groupe mobile.

5. Procédé suivant la revendication 4 (a), caractérisé en ce que la réduction est effectuée avec de l'hydrogène et un catalyseur au palladium dans un solvant convenable.

6. Procédé suivant la revendication 4 (a), caractérisé en ce que la réduction est effectuée avec de l'hydrure borosodique dans un solvant convenable.

7. Formulation pharmaceutique, caractérisé en ce qu'elle comprend un composé de formule (I) suivant la revendication 1 et un support acceptable du point de vue pharmaceutique pour celui-ci.

8. Formulation pharmaceutique suivant la revendication 7, sous forme de dose unitaire.

9. Procédé de production d'une formulation suivant la revendication 7, caractérisé en ce qu'on associe le composé de formule (I) avec un support.

10. Composé suivant la revendication 1, utile pour traiter le corps humain ou animal.